# FASCICULE DE BREVET EUROPEEN

(11) **EP 1 478 301 B1**
(45) Date de publication et mention de la délivrance du brevet: **14.04.2010**
(21) Numéro de dépôt: 03702268.8
(22) Date de dépôt: 27.02.2003
(51) Int. Cl.: A61C 8/00, A61C 1/14, A61B 17/16

(54) **OUTIL DE PREPARATION OSSEUSE UTILISABLE NOTAMMENT EN MEDICINE DENTAIRE ET DISPOSITIF POUR SA MISE EN OEUVRE**
INSTRUMENT ZUR VORBEREITUNG VON KNOCHENMATERIAL, VERWENDBAR INSBESONDERE IN DER ZAHNTECHNIK UND VORRICHTUNG ZU SEINER VERWENDUNG
BONE PREPARATION TOOL ESPECIALLY USEFUL IN DENTISTRY AND DEVICE FOR THE USE THEREOF

(30) Priorité: 27.02.2002 CH 340022002
(43) Date de publication de la demande: 24.11.2004
(73) Titulaire: Arsline S.A., 6833 Vacallo (CH)
(72) Inventeur: TURRI, Achille, CH-6834 Morbio Inferiore (CH)
(74) Mandataire: Störzbach, Michael Andreas
(86) Numéro de dépôt international: PCT/CH2003/000145
(87) Numéro de publication internationale: WO 2003/071978

(56) Documents cités:
- WO-A-01/85051
- CH-A- 410 276
- DE-A- 4 316 955
- DE-A- 19 732 983
- US-A- 5 437 675
- US-B1- 6 171 312

## Description

La présente invention porte sur un outil de préparation osseuse, en particulier un dilatateur utilisable notamment en médecine dentaire selon le préambule de la revendication indépendante 1, et sur un dispositif muni d'un outil de ce type se/on la revendication 20, pour on permetre une mise en oeuvre.

Le travail de préparation préalable à la pose d'un implant à insertion axiale chez un patient conduit-le-praticien spécialisé à réaliser dans la mâchoire de celui-ci un site (ou siège) osseux (appelé aussi siège de l'implant ou lit implantaire), apte à recevoir l'implant. Pour ce faire, il effectue dans le maxillaire un pertuis de diamètres variés se situant en général dans un intervalle allant de l'ordre de 3 à 6 mm, ce pertuis constituant en quelque sorte une alvéole artificielle.

Il existe actuellement deux méthodes de préparation de site osseux en vue de la pose d'implants: le plus souvent par forage dans l'os de la mâchoire (c'est-à-dire par perçage conventionnel entraînant un enlèvement de particules osseuses) et plus rarement par compression osseuse au pointeau (on parle couramment de technique de dilatation (du pertuis), la compression de la matière osseuse à la périphérie du pertuis résultant de cette dilatation produite par le pointeau). D'un point de vue pratique, la préparation du siège de l'implant peut se heurter dès le départ à un premier problème d'ordre physiologique. En effet, dans la zone où la pose de l'implant est prévue, la matière osseuse peut être insuffisante, soit en volume, eu égard aux dimensions transversales et/ou axiales, soit en densité, lorsqu'elle est constituée d'une spongieuse lâche (trabéculation avec larges lacunes). Notamment dans la seconde situation évoquée, la technique du forage est souvent inadaptée du fait que l'enlèvement de particules osseuses peut entraîner une insuffisance de structure osseuse et, partant, une retenue mécanique déficiente, donc compromettre la stabilité primaire de l'implant. Or, cette stabilité est, à côté d'une bonne résistance mécanique de l'implant, une condition strictement nécessaire pour l'ostéo-intégration physiologique, dudit implant.

C'est pourquoi, à l'heure actuelle, certains praticiens appliquent, lorsqu'ils sont confrontés à cette situation, un procédé de préparation osseuse consistant à dilater le site par compression du tissu osseux environnant qui devient ainsi plus compact, mécaniquement plus résistant et apte à une meilleure ostéo-intégration, cela au moyen de pointeaux cylindriques ou légèrement coniques. Pour ce faire, après avoir, le cas échéant, pratiqué un pertuis de départ de faible diamètre (de l'ordre de 2 mm) par forage à l'aide d'une fraise ou d'un foret usuel (forage pilote), le praticien place un premier pointeau de diamètre légèrement supérieur au diamètre de la fraise et tape sur ledit pointeau à l'aide d'un marteau, avec une force pouvant être assez conséquente, jusqu'à atteindre la profondeur voulue, puis effectue la même opération avec un deuxième pointeau de diamètre légèrement supérieur au diamètre du pointeau précédent, et ainsi de suite, en utilisant un jeu de pointeaux de diamètres croissants pour aboutir au pertuis de diamètre (et de profondeur) souhaités.

Bien entendu, les techniques de forage et de compression osseuse ne sont pas nécessairement exclusives l'une de l'autre, mais peuvent au contraire s'avérer complémentaires au cours de la préparation d'un même site osseux. En effet, il peut apparaître préférable ou même obligatoire, compte tenu de la dureté de l'os et/ou d'une possible variation de cette dureté au cours d'une opération, plutôt que de préparer un lit implantaire entièrement aux pointeaux, notamment de réaliser dans un premier temps plusieurs forages progressifs (par exemple jusqu'à 3.20 mm) suivis, dans un deuxième temps, d'au moins une opération de dilatation au(x) pointeau(x) jusqu'au diamètre final (par exemple de 3.50 mm).

La technique de compression osseuse au moyen des pointeaux utilisés actuellement présente une série de plusieurs inconvénients:
L'utilisation de tels outils est fortement traumatisante pour le patient. En effet, l'anesthésie étant locale, ce dernier, s'il ne ressent pas véritablement de douleur, perçoit tout de même la résonance des chocs dans sa tête.

Ne pouvant ressentir de façon suffisamment fine la résistance et la consistance de l'os, il est très difficile au praticien d'apprécier ces paramètres, donc de moduler l'état de dilatation dans un but d'optimiser la retenue de l'implant et sa stabilité primaire. Il ne peut non plus contrecarrer la tendance de déviation du pointeau de son axe de travail correct, inconvénient qui se manifeste en présence d'une corticale osseuse d'épaisseur asymétrique dans le sens transversal, comme c'est fréquemment le cas, par exemple, dans la zone des incisives et canines supérieures, où la corticale palatine est classiquement plus épaisse.

L'intensité des chocs provoqués par des frappes du marteau sur le pointeau, outre le fait qu'il est difficile de la doser correctement, est nécessairement variable. De plus, le praticien ne pouvant moduler avec suffisamment de précision la compression de la matière osseuse vers la périphérie du pertuis en fonction des paramètres anatomiques du moment, ne maîtrise pas donc non plus le calibrage du trou. Or, un parfait calibrage est une condition sine qua non pour une stabilité primaire optimale, elle-même préalable obligé pour la meilleure ostéo-intégration possible chez un patient donné.

Se greffant sur ces inconvénients, l'éventuelle non-homogénéité de la densité ou dureté osseuse (évoquée plus haut) le long de la paroi du pertuis peut conduire le praticien à devoir mettre en oeuvre, momentanément, un forêt usuel tranchant.

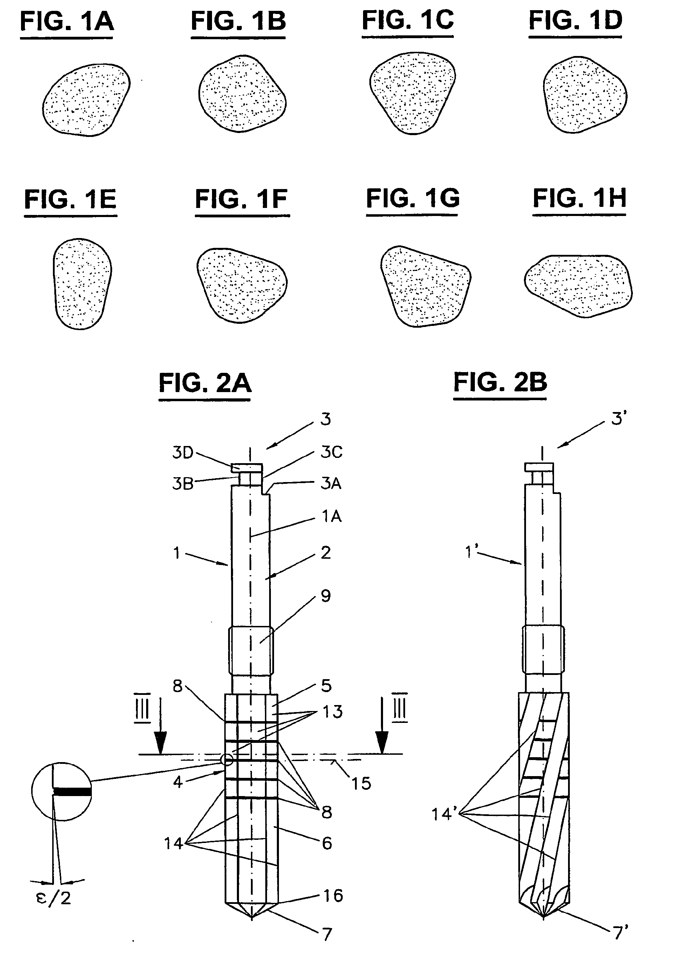

Par ailleurs, classiquement, la section droite des pertuis effectués en situation de densité osseuse insuffisante avec les pointeaux connus sont nécessairement circulaires, ces outils étant cylindriques, ou à la rigueur coniques. Mais d'autre part, les sièges des racines des dents naturelles (notamment les sections à la base de la couronne (ou au collet)) présentent des formes variées, c'est-à-dire des contours ou pourtours non réguliers et se démarquant plus ou moins sensiblement de la forme circulaire, comme le montrent schématiquement, à titre d'exemples, les figures 1 A (incisive centrale supérieure), 1 B (incisive centrale inférieure), 1 C (canine supérieure), 1 D (canine inférieure), 1 E (première prémolaire supérieure), 1 F (seconde prémolaire inférieure), 1 G (première molaire supérieure), 1 H (première molaire inférieure). Il apparaît qu'un siège osseux circulaire peut, pour la pose de certains implants, s'avérer tout à fait satisfaisant, voire indiqué (voir par exemple figure 1 F). En revanche, dans d'autres cas (voir par exemple les figures 1A, 1C, 1E, 1H), un pertuis de section circulaire reste un pis-aller tout autre qu'idéal, alors que le fait de se rapprocher au moins approximativement de la forme naturelle procurerait le double avantage d'un meilleur ancrage et d'une esthétique idéale de la suprastructure prothétique.

De plus, la forme ou le profil d'une racine, dans un plan axial, n'est pas davantage ou pas nécessairement bien cylindrique ou conique.

L'usage et la manipulation des pointeaux cylindriques apparaît donc grossière et peu appréciée, et l'on comprend que dans ces conditions un résultat optimum n'est pas assuré, tandis que les inconvénients rencontrés restreignent considérablement les limites de l'implantologie lorsque l'on se trouve face à une situation de densité osseuse insuffisante. Cette manipulation n'est d'ailleurs pas connue de tous les praticiens, et ceux qui la connaissent y font rarement appel, lui préférant le plus souvent la technique classique du forage, même lorsqu'ils sont confrontés à la situation critique précitée, ce qui peut mener à des résultats cliniquement décevants. Or le problème est d'autant plus critique que la technique du forage apparaît insuffisante, voire contre-indiquée dans de très nombreuses situations, puisque notamment les densités osseuses du maxillaire supérieur édenté sont, selon les statistiques, faibles à très faibles dans environ 80 % des cas (catégories de densité de l'os D3 et D4 selon Misch).

DE-A1-197,32,983 divulgue un outil pour effectuer un élargissement, ou plus précisément un écartement des segments d'une crête osseuse (on parle également de dislocation osseuse en bois vert ou Bone-Splitting) coupée le long de son axe longitudinal, pour augmenter le diamètre transversal, en vue d'une pose d'un implant. Le problème posé est d'éviter une fracture osseuse, la partie inférieure des deux tranches (ces dernières résultant de l'écartement) ne devant en aucun cas se séparer. Pour résoudre ce problème, l'outil proposé dans cette divulgation, qui met en application le principe de travail d'un levier d'expansion, présente une section cruciforme avec une "quille centrale" (10, 11) à bouts radiaux arrondis et, en direction axiale, sur une distance, une zone terminale légèrement conique avec une extrémité apicale axiale arrondie ou étagée. Pour le manoeuvrer, l'outil est inséré dans une poignée présentant un coude. L'opération de "Bone-Splitting", donc l'écartement des segments de la crête osseuse, se fait graduellement au moyen d'un jeu d'outils de largeurs croissantes (la largeur de la quille étant inférieure à la largeur de l'outil), chaque fois par mouvements rotatifs d'un angle déterminé et limité, de l'ordre de 45°. L'outil divulgué apporte une solution, parmi d'autres, à l'insuffisance en volume de la matière osseuse évoquée plus haut, même s'il apparaît à l'analyse qu'un risque d'enlèvement involontaire de matière osseuse n'est pas totalement écarté. En revanche, il ne constitue aucunement une réponse au problème de l'insuffisance en consistance de la matière osseuse et n'est pas apte à engendrer une compression de la structure maillée de l'os. Ce n'est pas là l'objectif recherché, et il n'en est d'ailleurs pas question dans DE-197,32,983.

Le document DE 43 16 955 A décrit un outil visant à fendre ou à écarter des parties de la crête osseuse, qui, de ce fait, ne répond pas au domaine de l'invention tel que défini dans le préambule de la revendication 1. Le document US 5,437,675 décrit un outil de compression osseuse, le compactage de l'os étant obtenu par un mouvement de translation dans le sens de l'axe dudit outil.

Le but de la présente invention est double, la première branche consistant à réaliser un outil de préparation osseuse plus particulièrement utilisable en implantologie dentaire permettant d'effectuer un véritable travail de compression de la structure alvéolaire osseuse, quelles que soient les différences quant aux duretés, densités et structures osseuses pouvant apparaître au cours de cette préparation, et qui ne soit pas affecté des inconvénients décrits plus haut.

Cette première branche du but est atteinte grâce à un outil de compression osseuse, ou dilatateur, répondant aux moyens définis dans la revendication indépendante 1, les revendications dépendantes portant sur des caractéristiques de réalisations préférées.

Observons dans ce contexte que l'outil revendiqué, au-delà des inconvénients exposés qu'il permet d'écarter, procure encore d'autres avantages d'importance qui ressortiront plus loin à la lecture de la description détaillée. Est à mentionner plus particulièrement l'avantage de nature fonctionnelle, à savoir une meilleure tenue de l'implant ou, en d'autres termes, un meilleur ancrage mécanique et son optimisation en toutes circonstances et conditions, y compris lors de circonstances changeantes où la densité osseuse locale est réduite, impossible d'atteindre avec les outils connus, la qualité de l'ancrage étant essentiellement fonction de l'interface os - implant.

Cette interface est elle-même fonction de deux paramètres.

Un premier paramètre porte sur la qualité de l'os, c'est-à-dire la densité de celui-ci. Grâce à l'outil, on parvient à améliorer la densité osseuse par compression. Par l'écrasement des lacunes osseuses, aussi bien la densité osseuse que la surface de contact avec l'implant sont augmentées. En l'occurrence, cette compression, outre le fait qu'elle est pratiquée avec un maximum de confort pour le patient, parce que tout "en douceur", est adaptée à l'individu, toutes les données spécifiques de la structure osseuse du site implantaire du patient étant exploitée au mieux.

Le second paramètre est fonction de l'ampleur de la surface de contact entre l'implant et la paroi osseuse du pertuis, celle-ci pouvant être influencée non seulement par la géométrie de la surface de l'implant et par son état microscopique plus ou moins rugueux, ce qui est commun, mais aussi et de plus - grâce au nouvel outil selon l'invention, par la forme macroscopique du pertuis réalisé. En effet, selon la forme ou la variante d'exécution de l'outil, il est possible d'exécuter un lit implantaire autre que circulaire - ce qui peut être avantageux, eu égard notamment à la localisation de l'implant et des caractéristiques anatomiques ou physiologiques du site osseux - par exemple en forme d'ovoïde (ou selon une courbe de Cassini) pour une localisation prémolaire, dont la surface de paroi est plus ample que celle d'une paroi cylindrique (à diamètre de base identique). La surface de contact sera donc augmentée, ou pour le moins sera moins réduite dans les cas où, pour une raison donnée, la profondeur de l'implant doit être relativement faible ou limitée.

En reproduisant ou en s'approchant mieux des formes et proportions des alvéoles naturelles, des formes d'exécution spécifiques de l'outil de préparation osseuse permettent de réaliser des poses d'implants de dimensions idéales. Ainsi ces derniers sont-ils aptes à supporter des suprastructures prothétiques fixes aux qualités sensiblement supérieures en comparaison avec les prothèses fixes actuelles (étant rappelé qu'à l'heure actuelle, la forme des racines artificielles reste sensiblement éloignée des différentes racines naturelles), cela aussi bien sur les plans mécanique (un implant de forme ovoïde, par exemple, résiste mieux aux forces de torsion qu'un implant de forme circulaire), physiologique et esthétique (suppression de niches ou de vides, pose d'éléments prothétiques (couronnes sur implants) qui sont de véritables répliques des dents naturelles).

En aval, en permettant d'élaborer des alvéoles aux formes spécifiquement adaptées, c'est-à-dire proches des alvéoles naturelles, l'outil de préparation osseuse, de surcroît de caractère polyvalent, ouvre la voie à la création d'une nouvelle génération d'implants de formes également anatomiques.

La seconde branche du but est évidemment intimement liée à la première branche précitée, s'agissant d'une mise en oeuvre du dilatateur ou plus généralement de l'outil de pénétration osseuse au moyen d'un dispositif muni de cet outil.

Un tel dispositif n'est pas connu à ce jour. US-6,171,312 divulgue des formes de réalisation de dispositifs de mise en oeuvre d'un ostétome,. Ces dispositifs connus sont destinés à imprimer audit ostéotome essentiellement un mouvement axial de va-et-vient, de course définie. Selon une variante, un mécanisme permet un entraînement en rotation d'amplitude limitée ("minimal rotation"), en tout état de cause en combinaison avec le mouvement de translation.

L'invention concerne donc également un dispositif nouveau et original selon les moyens définis dans la revendication 20, permettant d'assurer une mise en oeuvre optimale de l'outil de préparation osseuse commenté plus haut. Les revendications dépendantes 21 et suivantes portent sur des réalisations particulières de ce dispositif, dont le fonctionnement peut être soit manuel, soit automatique.

Différentes formes de réalisations du dilatateur et du dispositif pour manoeuvrer ce dernier vont être décrites ci-après, à titre d'exemples non limitatifs, à l'appui des dessins annexées, où
les figures 1A à 1H (déjà commentées) montrent des formes de sections de racines de dents à la base de la couronne (proche du collet),
les figures 2A et 2B sont des vues de face (plans axiaux orthogonaux) d'une forme de'réalisation du dilatateur, selon deux variantes,
la figure 3 est une coupe selon la ligne III - III de la figure 2,
les figures 4 à 7 représentent des variantes d'une première forme d'exécution de la section droite de la-partie active du dilatateur,
la figure 8 représente une variante d'une deuxième forme d'exécution de la section droite de la partie active du dilatateur,
les figures 9A et 9B sont des schémas comparatifs (pointeau traditionnel / dilatateur selon l'invention),
la figure 10 montre en perspective une forme d'exécution d'un dispositif d'actionnement automatique d'un dilatateur,
la figure 11 est une coupe de cette exécution de dispositif automatique selon un plan (P) de la figure 10,
la figure 12 montre en perspective le montage des différents organes du dispositif d'actionnement, boîtier enlevé,
les figures 13 et 14 montrent chacune, en perspective, la coopération de deux organes entre eux, réducteur et percuteur, ce dernier en position haute et basse respectivement,
la figure 15 est une vue en perspective d'une forme de réalisation d'un dispositif manuel,
les figures 16 à 19 sont des schémas explicatifs du fonctionnement de cette forme de réalisation de dispositif manuel,
la figure 20 est une vue en perspective (partielle) d'une autre forme de réalisation d'un dispositif manuel,
la figure 21 est une vue en coupe du dispositif montré à la figure, selon un plan de symétrie, et
la figure 22 une autre vue en perspective (partielle) de cette autre forme de réalisation d'un dispositif manuel,
étant noté que les échelles de représentation des différentes figures ne sont pas uniformes.

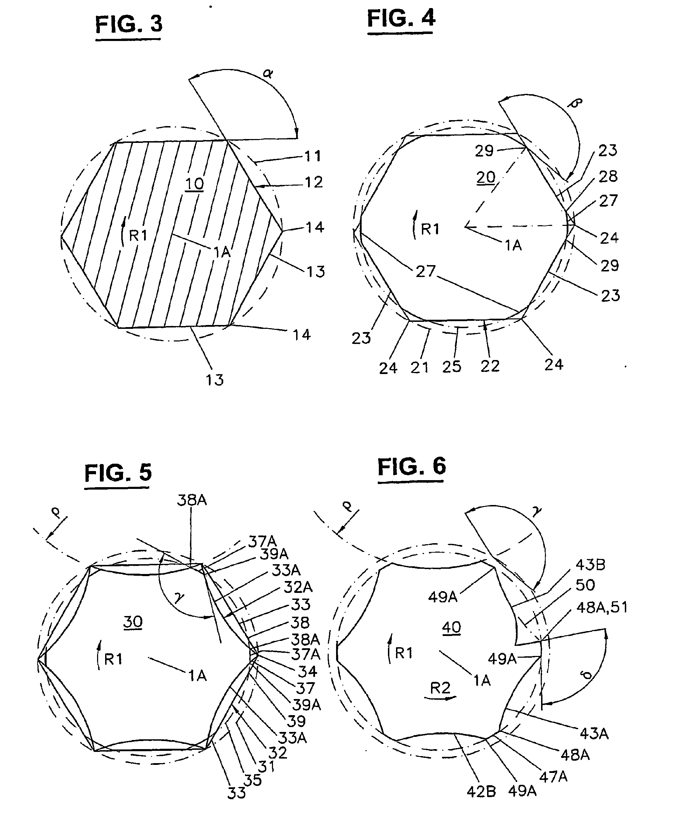

Une réalisation simple d'un dilatateur 1 est représentée en vue de face à la figure 2A et en coupe selon une ligne III-III de cette dernière à la figure 3. Ce dilatateur, d'axe 1 A, présente une tige 2 avec une queue de tige 3 comportant un épaulement 3 A, une gorge 3 B, un méplat 3 C et un chapeau 3 D, cette tige se prolongeant d'une partie active 4 pour le façonnement du pertuis, c'est-à-dire du siège osseux ou site implantaire. La partie 4 comporte des faces 13 séparées par des arêtes 14, droites. Selon une variante montrée à la figure 2B, le dilatateur 1' pourraient présenter des arêtes 14' hélicoïdales. La partie active 4 se compose avantageusement d'une sous-partie arrière 5 et d'une sous-partie avant 6 dont l'extrémité présente une partie apicale ou tête d'attaque 7 (7' selon la variante de la figure 2B). La sous-partie 6 s'étendant entre un plan 15 et la base 16 de la tête 7 accuse avantageusement une très légère conicité ε, par exemple de l'ordre de 1° (voir agrandissement de la partie entourée d'un cercle). Cette conicité procure un double avantage. En effet, lors de l'élaboration d'un siège osseux, les insertions successives d'un jeu de dilatateurs 1 aux sections radiales à dimensions croissantes se font en tout douceur, puisque la section de la partie active d'un dilatateur à la base 16 est dimensionnée de telle sorte qu'elle correspond au maximum à la dimension de la section de la partie active du dilatateur 1 précédemment utilisé au niveau du plan 15. Elle assure ensuite une tenue plus serrée de l'implant, donc de meilleure qualité. En pratique, la longueur de la sous-partie tronconique 6 est avantageusement de l'ordre de dix millimètres, ce qui correspond à la profondeur moyenne des sièges osseux pratiqués pour la pose d'un implant (étant précisé que le terme tronconique ne signifie pas ici que la section de la sous-partie 6, et plus généralement de la partie 4, soit circulaire (voir infra)). La partie active 4 est de préférence pourvue de repères de profondeur 8, tandis que la tige peut comporter, dans sa partie avant (en regardant dans la direction {tige - partie active}, un filetage 9 prévu pour coopérer avec un dispositif de sécurité (décrit dans WO 00/74585, du même inventeur) rendant impossible l'introduction du dilatateur dans le pertuis au-delà d'une profondeur limite P préalablement déterminée par le praticien.

Toutefois, comme on l'a vu plus haut, même un profil conique du dilatateur, dans le plan axial, peut ne pas s'avérer idéal, car pouvant s'éloigner trop du profil naturel et individuel. Aussi est-il possible de prévoir des dilatateurs dont le profil de la partie 4 ou du moins de la sous-partie 6 revêt toute autre forme adéquate. Ainsi, on peut envisager, entre autres exemples, un profil courbe (par exemple en forme de flamme inversée), ou mixte, c'est-à-dire droit sur une longueur et courbe sur une autre, ou encore étagé, par succession d'épaulements et de tronçons cylindriques ou coniques de dimensions décroissantes en direction de la tête 7.

Par la suite, on désignera par dilatateur, avec ou sans la référence 1, tout dilatateur selon l'invention, quelles que soient les formes (ou pourtour ou contour, ces mots étant entendus non un sens métrique mais de forme) de la section droite et/ou du profil de sa partie active 4.

De manière générale, comme on le comprendra aisément à la lecture du mode d'actionnement du dilatateur, la section droite de la partie active 4 de celui-ci peut présenter, à l'inverse des pointeaux connus, toute forme autre que circulaire. Par convention, le caractère non circulaire est défini ici par la fait que l'écart relatif entre le diamètre du cercle passant par le point de la section ou l'ensemble de points le plus éloigné de l'axe de rotation et le diamètre du cercle passant par le point de la section ou l'ensemble de points le plus rapproché dudit axe est égal ou supérieur à 0.5 %. La section du dilatateur, c'est-à-dire de sa partie active, est définie en fonction et résulte de la combinaison, d'une part, de la forme (ou pourtour) souhaité de la section droite du siège osseux et, d'autre part, du mouvement ou de la combinaison de mouvements qu'il est prévu d'imprimer au dilatateur.

Selon une première forme d'exécution, elle-même aux multiples variantes possibles, le dilatateur permet la réalisation de pertuis ou sièges implantaires dont la section est circulaire. Une forme avantageuse de section de la partie active du dilatateur, parmi une infinité de possibilités, consiste en un polygone avantageusement convexe et régulier, ou en une figure ayant une allure générale polygonale, ce polygone pouvant présenter ou non des particularités de forme spécifiques, les angles d'attaque (voir plus loin) étant de préférence obtus.

Ainsi, selon une première variante 10 représentée à la figure 3, la section droite (ou radiale) du dilatateur est hexagonale (par convention, on désignera une variante par la même référence que celle de la section qui en constitue une particularité). L'hexagone régulier de contour 12 est inscrit dans un cercle 11 (dessiné en trait mixte fin). Pour simplifier, les six côtés et les six sommets portent les mêmes références que les faces et les arêtes correspondantes de la partie active 4 (figure 1), soit 13 et 14 respectivement (simplification qui sera adoptée dans la description ultérieure d'autres variantes). Selon une exécution non représentée, chacun des sommets 14 peut être émoussé, par exemple par formation d'un léger arrondi par polissage, de sorte à supprimer le risque d'un effet tranchant (ou pour le moins à minimiser ce risque) des arêtes correspondantes. Est encore matérialisé sur la figure 3 un angle α, qu'on appellera angle d'attaque (égal ici à 120°) en imaginant une rotation dans le sens R 1 (sens horaire). (Nota: dans cette figure 3 ainsi que dans celles qui suivent, les éléments identiques ne sont pas nécessairement tous référencés).

Selon une deuxième variante montrée à la figure 4, analogue à la variante 10, le pourtour 22 de la section droite 20 (non hachurée) de la partie active du dilatateur résulte d'un cercle 25 et d'un hexagone régulier (de côtés 23 et de sommets 24) inscrit dans un cercle 21 concentrique au cercle 25 (étant observé que certaines particularités de forme propres à cette variante 20 qui se dégagent à la lecture de ladite figure, ni celles des variantes suivantes qui vont être décrites plus loin, ne sont pas représentées dans la figure 2). Ainsi, les six sommets de la section 20, au lieu d'être vifs comme dans la variante 10, sont ici arrondis, en l'occurrence constitués d'arcs de cercle 27 d'extrémités 28, 29, séparant les côtés 23. Compte tenu du faible diamètre du cercle 25 (le diamètre extérieur le plus grand variant en pratique, pour un jeu de dilatateurs donné, de 3 à 6 mm environ), ces arcs de cercle sont assimilables à des méplats, leur longueur étant avantageusement de l'ordre de un à trois dixièmes de mm. En comparaison avec la variante 10, cette construction diminue l'effet tranchant (évoqué plus haut) d'une arête vive du polygone et augmente l'angle d'attaque β (angle au sommet formé à chaque extrémité 29 et dont les deux côtés sont le côté 23 et la tangente audit point 29 au cercle 25) (β > α, rotation dans le sens de la flèche R1).

Une section 30 (non hachurée) représentée à la figure 5 constitue une troisième variante, analogue à la variante 20 de la figure 4, à une particularité de forme près, explicitée ci-après. Un contour initial 32 résulte d'un hexagone régulier inscrit dans un cercle 31, de côtés 33 et de sommets 34, et d'un cercle 35 concentrique au cercle 31, de sorte à obtenir six sommets arrondis, c'est-à-dire constitués d'arcs de cercle 37 d'extrémités 38, 39, séparant les côtés 33). En partant de cette section de départ, les côtés 33 sont incurvés vers l'axe du dilatateur pour former des côtés concaves 33 A, par exemple par usinage des faces respectives de la partie active 4 suivant un rayon de courbure p défini. Un contour 32 A est ainsi formé de six côtés concaves 33 A séparés de sommets arrondis 37 A assimilables là aussi à des méplats d'extrémités 38 A, 39 A. Ce pourtour s'apparente à une hypocycloïde modifiée à six côtés et à six sommets arrondis. L'angle d'attaque au sommet 39 A (rotation dans le sens de la flèche R1) est désigné par γ (angle formé à chaque sommet 39 A, les côtés étant constitués des tangentes en 39 A au cercle 35 et au cercle de rayon p). On observe qu'à chaque sommet, la longueur de l'arc de cercle 37 A est sensiblement inférieure à celle de l'arc de cercle 37, cette réduction étant bien entendu fonction de la valeur choisie pour le rayon de courbure ρ.

Une quatrième variante (section 40 non hachurée) représentée à la figure 6 est analogue à la variante 30 de la figure 5, à la différence près que le pourtour (référencé ici 42 B) quasi hypocycloïdal est modifié, l'un des côtés (référence 43 B) présentant une entaille 50, de sorte à former avec l'une des extrémités 48 A d'un arc de cercle ou méplat 47 A (extrémités 48 A, 49 A) un sommet pointu 51 dont la succession sur la partie active 4 constitue une arête tranchante d'angle d'attaque δ dans le sens de rotation symbolisé par la flèche R2, l'angle d'attaque γ aux points 49 A (voir aussi figure 5) restant inchangé. Bien entendu, il est possible de prévoir plusieurs arêtes tranchantes, plusieurs côtés 43 B, voire tous, pouvant être façonnés de sorte que plusieurs sommets comportent une pointe 51. Cette variante présente l'avantage de dispenser le praticien de changer d'outil au cours d'une opération de compression osseuse, comme on le précisera plus loin dans la partie consacrée aux dispositifs de mise en oeuvre du dilatateur.

On conçoit qu'un grand nombre d'autres formes de sections peuvent être réalisées. Notamment le polygone de départ pourrait être un pentagone, un octogone, etc., tandis que les côtés peuvent obéir à des dessins particuliers.

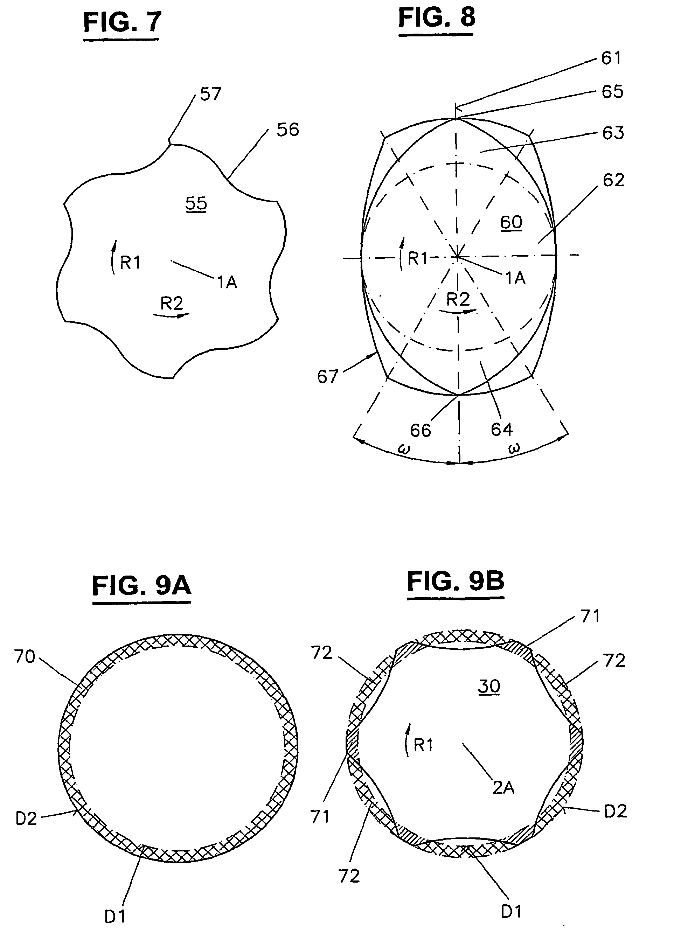

A titre d'exemple, la figure 7 représente une section 55, d'allure générale hexagonale, dont les côtés 56 correspondent à un genre de sinusoïde et dont tous les sommets 57 (c'est-à-dire toutes les arêtes correspondantes) présentent l'avantage de pouvoir exercer une double fonction de compression et de coupe (voir infra).

Selon une autre forme d'exécution, également aux multiples variantes possibles, le dilatateur est conçu de sorte à pouvoir réaliser un siège implantaire dont la section reproduit ou s'approche au moins approximativement de la section d'une alvéole naturelle déviant sensiblement de la forme circulaire (voir figure 1).

La figure 8 montre un dilatateur 1 de section 60 (non hachurée), d'axe de symétrie 61, qui comporte une âme circulaire 62 et au moins une protubérance (la forme serait approximativement celle d'une came), selon l'exemple dessiné deux protubérances 63, 64 symétriques par rapport à l'axe 1 A (forme approximative d'un double ovoïde). Ces protubérances à flancs courbes présentent des extrémités 65, 66 (qui peuvent être arrondis par meulage). Par un actionnement adéquat du dilatateur, comme on le verra par la suite, on obtient un siège implantaire de pourtour 67, correspondant grossièrement ici à une courbe de Cassini et proche du contour d'une alvéole naturelle selon la figure 1 H. On conçoit aisément à partir de cet exemple que d'autres formes de sections peuvent être réalisées pour façonner un lit implantaire dont le pourtour reproduit le pourtour d'une alvéole naturelle non circulaire ou pour le moins s'en rapprochera au mieux (par exemple section de forme ovoïde simple). Ces dilatateurs à sections spécifiques (dont on peut prévoir là aussi des jeux de dimensions croissantes) peuvent le cas échéant être avantageusement mis en oeuvre après une première élaboration d'un pertuis circulaire au moyen de dilatateurs selon la première forme d'exécution (figures 3 à 7).

On va exposer maintenant le mode de mise en oeuvre des dilatateurs selon l'invention permettant le façonnage d'un site implantaire.

Comme cela a été dit plus haut, après une éventuelle élaboration, dans un premier temps, d'un pertuis pilote de faible diamètre (2.0 mm par exemple), à une profondeur P voulue, par forage usuel, le siège implantaire est dilaté progressivement grâce à un jeu de dilatateurs de dimensions croissantes, jusqu'à achèvement de l'élaboration du siège osseux final prévu pour la pose de l'implant, l'incrément étant en général de l'ordre de 0.10 à 0.25 mm, étant entendu que le praticien adaptera celui-ci aux circonstances momentanées (par exemple, choix d'un incrément plus faible en présence d'os dur ou lorsque l'on se rapproche du diamètre final prévu pour le siège).

Chaque dilatateur 1 d'un jeu est soumis à deux types de mouvements, à savoir un mouvement de translation, le long de l'axe 1A, et un mouvement de rotation dans le sens horaire et/ou anti-horaire.

Selon un processus, ces deux types de mouvements, translation et rotation, sont, pour engendrer la compression de la paroi osseuse, exercés en deux phases successives.

Une première phase consiste à introduire axialement un dilatateur 1 dans le pertuis, par chocs axiaux répétés transmis à la tige 2, jusqu'à ce qu'une profondeur définie ait été atteinte (celle-ci peut être égale ou inférieure à la profondeur finale P du siège implantaire, cette dernière pouvant être atteinte de proche en proche). Cette première phase engendre une première compression de matière osseuse, très limitée. On comprend à l'appui des figures 9A et 9B que, par rapport aux dilatateurs circulaires traditionnels, l'introduction avec un dilatateur selon l'invention se fait en douceur, puisque la matière déplacée / comprimée est considérablement plus réduite. En effet, pour étendre le siège implantaire d'un diamètre D1 à un diamètre D2 avec la méthode traditionnelle des pointeaux cylindriques, la quantité ou le volume de matière osseuse qu'il faut comprimer au moyen d'un pointeau de diamètre D2 correspondant à la surface hachurée 70 multipliée par la profondeur P ou momentanément choisie du pertuis, cela par des frappes axiales nécessairement de forte intensité. En comparaison (voir figure 9B), la quantité ou le volume de matière osseuse à comprimer lors de l'introduction axiale d'un dilatateur selon la variante 30 par exemple, correspond à la somme des aires 71 multipliée par la même profondeur, somme qui est très inférieure à l'aire 70. L'inconfort que produit chez le patient la préparation osseuse du siège implantaire est ainsi extrêmement réduit. D'autre part, le praticien contrôle parfaitement et aisément l'avancée axiale du dilatateur.

Une seconde phase consiste à soumettre ce même dilatateur à une rotation dans un sens R 1 (indiqué dans les figures 3-8 et 9B). C'est au cours de cette phase qu'a lieu la quasi totalité de l'opération de compression, le dilatateur chassant et simultanément comprimant vers la périphérie du siège la matière osseuse dont les mailles vont être resserrées en douceur, cela sans enlèvement de parties osseuses. A la figure 9B, la quantité de matière osseuse comprimée correspond à la somme des surfaces 72 multipliée par la profondeur P (ou la profondeur momentanée) du pertuis. Cette phase de compression, du fait qu'elle se déroule sans frappes sur le dilatateur, n'engendre pas d'inconfort particulier chez le patient. L'ampleur angulaire de la rotation ou le nombre de tours du dilatateur sera décidé cas par cas par le praticien, l'angle minimum étant en toute hypothèse dicté par le choix de la section de la partie activé du dilatateur (ainsi, en se référant par exemple à la variante 9B, le praticien fera tourner le dilatateur d'un angle de 60° au moins, de sorte à obtenir la forme circulaire du siège implantaire), et/ou par la section que l'on vise à obtenir pour le siège osseux.

Ainsi, en se référant à la figure 8, la compression de la paroi osseuse du pertuis ne procède pas de la seule rotation dans le sens horaire R1, mais par l'exercice de mouvements oscillants d'angle ω dans les sens R1 et R 2. Eu égard à la section 60 de l'outil évoquée plus haut, on aboutit ainsi au siège implantaire de pourtour 67, proche de la forme d'une alvéole naturelle selon la figurer 1 H.

Outre la compression de la matière osseuse qu'il provoque, le mouvement de rotation permet de moduler finement la compression en fonction de la qualité (variable) de l'os et, partant, de maîtriser le calibrage du siège. En particulier, lorsque l'os est spongieux, le praticien peut limiter la rotation au minimum, tandis que si l'os est dur, donc aussi plus élastique, il peut décider d'imprimer au dilatateur un tour ou même plusieurs tours complets, optimisant ainsi la qualité de la paroi du siège osseux tout en facilitant l'extraction du dilatateur hors dudit siège.

Lorsque le dilatateur comporte au moins une arête tranchante, (voir les figures 6 et 7, arêtes 51 et 57 respectivement), l'entraînement en rotation dans le sens anti-horaire R2 lui fera exercer une fonction de coupe. Telle rotation peut être d'amplitude limitée ou être effectuée sur un ou plusieurs tours. De plus, elle peut s'accompagner ou non d'un mouvement en translation. L'emploi d'un dilatateur apte à cumuler les fonctions de compression de la paroi osseuse et de coupe peut s'avérer utile en particulier si une progression du dilatateur en direction axiale se heurte à des difficultés, dues par exemple à la présence de zones osseuses plus denses que celles traversées jusque-là au cours de l'insertion du dilatateur vers le fond du site osseux, ou si une différence de densité osseuse tend à faire dévier le dilatateur de son axe de travail correct (à raison de la présence, par exemple, d'une corticale osseuse d'épaisseur asymétrique dans le sens transversal, comme cela se rencontre fréquemment lors de l'élaboration de sites pour les incisives et canines supérieures).

Selon un autre processus, les deux types de mouvements, translation et rotation, sont, pour engendrer la compression de la paroi osseuse, exercés concomitamment.

Bien entendu, dans ce cas, il est préférable que les arêtes du dilatateur, au lieu d'être droites comme représentées à la figure 2, soient au moins légèrement hélicoïdales. Cette manière de procéder facilite alors la pénétration de l'outil dans le pertuis, puisqu'il est soumis à une force de traction ayant tendance à le faire progresser vers l'intérieur du site osseux. En fait, le travail de compression résultant de la première phase du premier processus (compression que l'on peut qualifier d'auxiliaire, voir supra), et les frappes axiales y liées, d'impulsion certes négligeable, en comparaison de celle créée par les outils de l'art antérieur, est entièrement éliminé. En d'autres termes, tout se passe comme si les deux phases dudit premier processus étaient réunies en une seule, compression "auxiliaire" d'une part, et compression proprement dite engendrée par la seule rotation d'autre part, ayant lieu en même temps. Aussi l'éventuel inconfort rémanent que lesdites frappes pourraient laisser subsister se trouve totalement annihilé.

Enfin, en ce qui concerne la partie apicale ou tête d'attaque 7; 7' (cf. figures 2A et 2B), celle-ci peut être conformée de sorte à exercer, ou, à l'inverse, à ne pas exercer une action coupante, toutes combinaisons (au nombre de quatre) étant possibles: action coupante avec la seule rotation R1; action coupante avec la seule rotation R2; action coupante aussi bien avec la rotation R1 qu'avec la rotation R2; aucune action coupante, ni avec la rotation R1, ni avec la rotation R2.

Bien entendu, selon un mode de réalisation des exemples décrits, les actions exercées par la partie active du dilatateur par rapport aux sens de rotation pourraient être inversées, une rotation dans le sens R1 engendrant alors une coupe, si l'outil comporte au moins une arête tranchante (ou une compression à défaut d'une telle arête), et une rotation dans le sens R2 provoquant une compression. Cette inversion est applicable de manière analogue à la partie apicale.

Quant au choix de la section elle-même du dilatateur, il est fonction de la pondération de différents paramètres:
notamment intensité des chocs axiaux pour la pénétration axiale du dilatateur dans le pertuis (si l'on opte pour l'application du premier processus explicité), risque d'arrachage de matière osseuse, angle d'attaque, quantité de matière osseuse à compresser lors de l'avancée axiale du dilatateur. Ainsi, la pénétration axiale puis la rotation du dilatateur se feront-elles plus aisément avec un dilatateur selon la variante 10 qu'avec un dilatateur selon la variante 20, la surface et le volume de matière osseuse déplacée étant inférieurs avec les arêtes 13 qu'avec les méplats 27. En revanche, le risque d'enlèvement intempestif de matière osseuse, notamment lors de l'introduction axiale du dilatateur, risque d'être légèrement plus grand avec un dilatateur de section 10 qu'avec un dilatateur de section 20. La forme quasi hypocycloïde des sections 30, 40 permet de pallier l'inconvénient mentionné à propos de la section 20, en diminuant davantage encore la quantité de matière osseuse à comprimer lors du mouvement axial, l'angle d'attaque restant relativement grand. C'est pourquoi, une section du genre de celle représentée aux figures 5 et 6 constitue un compromis très satisfaisant: réduction considérable de l'intensité des chocs axiaux et excellente maîtrise de la modulation de la compression de la matière osseuse lors de la rotation du dilatateur (sens R 1). Ces considérations valent également, du moins pour partie, lorsqu'il est envisagé d'appliquer le deuxième processus décrit.

Le dilatateur devant être soumis, pour réaliser un siège implantaire défini, à des mouvements définis, l'homme du métier est confronté au problème des moyens permettant d'assurer ces derniers. C'est l'objet du deuxième volet de l'invention, qui porte sur des dispositifs nouveaux dont quelques formes d'exécution vont maintenant être décrites, à titre d'exemples non limitatifs, ces dispositifs pouvant être classés grossièrement en deux catégories, une première concernant des dispositifs automatiques, une autre des dispositifs manuels.

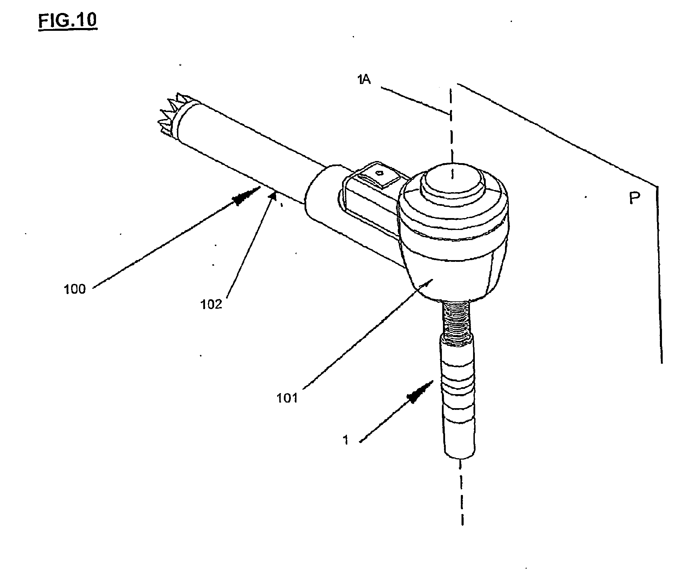

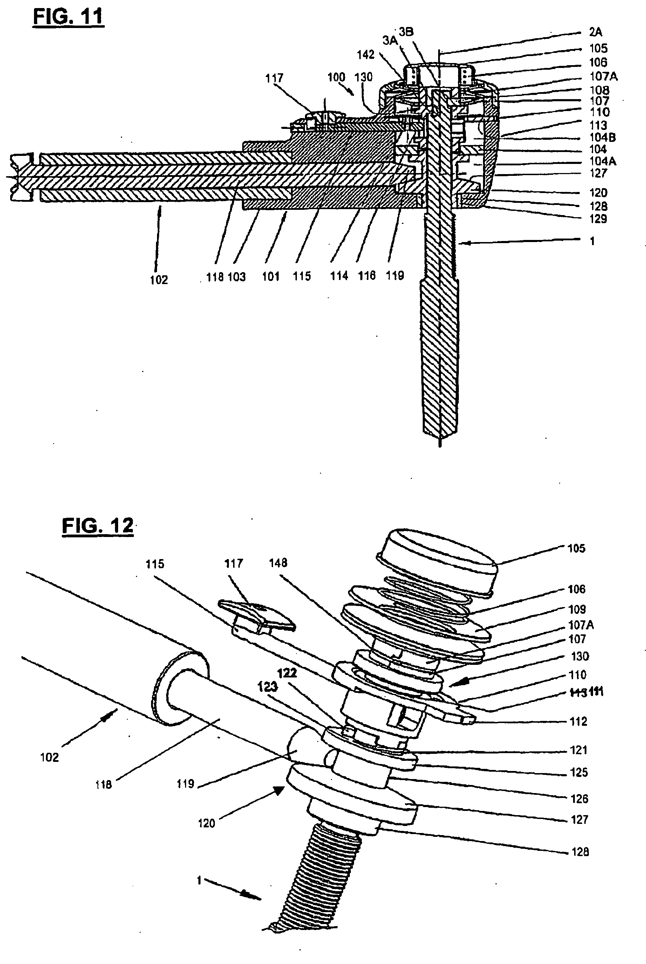

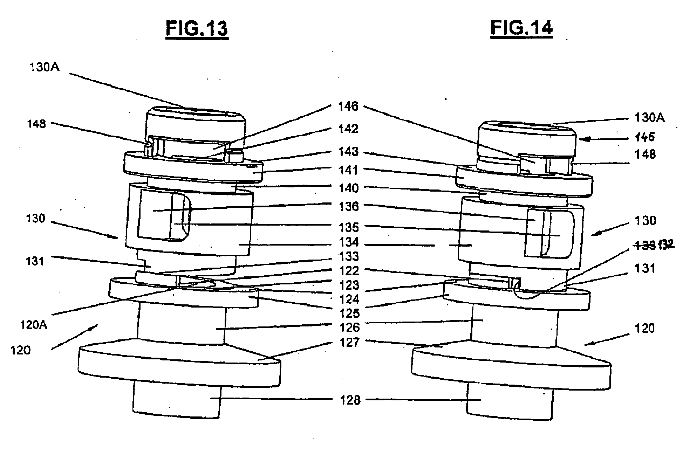

Entrant dans la première catégorie invoquée, une réalisation avantageuse d'un dispositif automatique 100 est représentée en perspective à la figure 10 et en coupe à la figure 11 selon un plan de symétrie P (figure 10) passant par l'axe 1 A se confondant avec l'axe de même référence du dilatateur 1. Les figures 12, 13 et 14 montrent à plus grande échelle les différents organes constitutifs essentiels que l'on aperçoit à la figure 11.

Le dispositif 100 se présente sous la forme générale connue d'une tête de contre-angle 101 située dans le prolongement d'un manche 102 et dans laquelle est enfiché un dilatateur, notamment un dilatateur 1 selon l'invention. Cette tête comporte un boîtier 103 abritant, dans deux logements 104 A (logement inférieur), 104 B (logement supérieur) séparés par une cloison 104 à ouverture centrale (non référencée), un mécanisme assurant notamment les fonctions d'un mandrin, soit le blocage et l'entraînement du dilatateur, un élément au moins du mécanisme étant soumis à l'action d'un arbre de transmission 118 sur l'extrémité duquel est fixé un pignon 119.

Le mécanisme permet d'actionner le dilatateur 1 aussi bien en translation, c'est-à-dire axialement, qu'en rotation, dans le sens horaire et dans le sens anti-horaire. A cette fin, il comporte essentiellement, selon un exemple de moyen de translation, un percuteur 130 agencé au-dessus d'un organe d'entraînement en rotation 120. Ces deux éléments 120, 130 coopèrent l'un avec l'autre et forment un canal 120 A, 130 A destiné à recevoir le dilatateur 1.

Le dilatateur 1 introduit dans le canal 120 A, 130 A est rendu solidaire du percuteur 130 et peut être libéré de celui-ci grâce à un système de verrouillage / déverrouillage classique connu comprenant une touche de décrochement 105 se prolongeant d'une patte (non représentée) et soumise à l'action d'un ressort de décrochement 106, un verrou 107 pourvu d'une aile 107 A, et un ressort 108 de retenue de verrou logé dans une rainure 148 du percuteur 130 (figure 12) et ceinturant ce dernier ensemble avec ledit verrou 107. Pour le verrouiller, le dilatateur est introduit dans le canal 120 A, 130 A en même temps qu'une pression est exercée sur la touche de décrochement 105 contre la force du ressort 106, la patte précitée dégageant ainsi le verrou 107 en arrière du prolongement du canal 130 A par rapport à l'axe 1 A. Lorsque l'épaulement 3 A de la tête 3 du dilatateur vient buter contre un talon 142 du percuteur 130, la pression sur la touche 105 est relâchée, le verrou 107 s'introduisant alors dans la rainure 3 B du dilatateur 1 sous l'action du ressort de retenue 108 pour assurer le blocage dudit dilatateur. Le déverrouillage du dilatateur 1 procède de l'opération inverse, une pression sur la touche 105 ayant pour effet de dégager le verrou 107 de la rainure 3 B du dilatateur 1 et donc de libérer ce dernier.

L'organe d'entraînement en rotation 120 présentant l'alésage 120 A, est formé d'un réducteur logé dans l'espace inférieur 104 A et remplissant en même temps une fonction de crapaudine libre en rotation. Le réducteur 120 comprend une couronne dentée conique 127 en prise avec un pignon 119, ce réducteur démultipliant la vitesse de rotation de l'arbre de transmission 118 et multipliant ainsi le couple transmis au percuteur 130. La partie inférieure du réducteur 120 présente une portée de guidage 128 guidée dans un palier 129. Au-dessus de la couronne 127 s'étend une portée 126 assurant un dégagement suffisant au pignon 119 et s'ouvrant sur une embase 125. La face supérieure de cette dernière présente un épaulement 124 s'appuyant sur la cloison 104 par l'intermédiaire, par exemple, d'un joint tournant (non représenté), et une rampe 121 hélicoïdale s'amorçant après un méplat 123, selon l'exemple sur 360° (y compris la partie plate 123), la partie terminale de la rampe formant une face d'appui 122.

L'organe d'entraînement en translation 130 (en l'occurrence le percuteur) présentant l'alésage 130 A est formé d'une pièce cylindrique étagée logée dans l'espace supérieur 104 B. La partie inférieure de cet organe 130 comporte une rampe 131 coopérant avec la rampe 121 du réducteur et présentant une forme complémentaire à cette dernière (pente hélicoïdale s'amorçant après un méplat 133, angle de 360° (y compris la partie plate 133, la partie terminale formant une face d'appui 132). S'étendent au-dessus de cette rampe 131, une partie centrale 134, un collet 141 séparé de la partie centrale 134 par une gorge 140, et une partie terminale 145 s'élevant de la face supérieure 143 du collet 141. La partie centrale 134 comporte deux ouvertures de guidage ou fentes 135 droites diamétralement opposées dont la hauteur est égale au moins au double de la hauteur de la face d'appui 122 du réducteur (ou 133 de percuteur) et dont le flanc gauche 136 chaque fois est arrondi. Au niveau du collet 141 s'avance dans le canal 130 A un talon 142 formant butée à l'épaulement 3 A du dilatateur. La partie terminale 145 présente une ouverture 146 dans laquelle peut s'introduire le verrou du dilatateur 107, ainsi qu'une rainure 148 dans laquelle prend place le ressort de retenue 108.

Autour de la partie 145 est agencé un organe élastique 109, plus particulièrement des disques ressorts ou ressorts Belleville dont les bords inférieur et supérieur (non référencés) prennent appui sur la face supérieure 143 du collet 141 et sur une coiffe (non référencée) du boîtier 103 respectivement. L'organe élastique 109 travaillant à la compression (donc exerçant une force contraire à une force de compression agissant sur lui), appuie constamment le percuteur contre le réducteur 120.

Une extrémité 116 d'un bras de sélection 115 peut être engagée indifféremment dans l'une ou l'autre des fentes 135 ou dégagé de ces ouvertures par l'action d'un bouton de sélection 117. Le bras 115 est disposé de telle sorte qu'une coulisse 110 dont il est solidaire par l'intermédiaire d'une goupille 114 se trouve au niveau de la gorge 140 du percuteur lorsque celui-ci est en position basse (voir plus loin). La coulisse est pourvue de protubérances intérieure 111 et extérieure 112 opposées l'une à l'autre, la protubérance intérieure 111 pouvant venir se loger dans la gorge 140 et la protubérance extérieure 112 dans une rainure 113 aménagée dans le boîtier.

Le fonctionnement du dispositif 100 est le suivant: Lorsque l'arbre de transmission 118 est actionné, son mouvement de rotation est transformé de telle sorte que le dilatateur 1 puisse être entraîné en translation, c'est-à-dire axialement en soumettant celui-ci à des mouvements de va-et-vient selon l'axe 1 A, et/ou en rotation.

Selon l'exemple, le mouvement en translation s'accompagne d'un effet de percussion sur le dilatateur 1. Pour provoquer cet effet, l'extrémité 116 du bras de sélection est engagée dans l'une des fentes 135 et bloque ainsi le degré de liberté en rotation du percuteur 130. L'arbre de transmission 118 entraîne le réducteur 130 en rotation dans le sens indiqué par la flèche R 1 (sens horaire). En imaginant un point de départ où le méplat 133 de la rampe 131 du percuteur 130 est en appui contre le méplat 123 de la rampe 121 du réducteur 120 et les faces respectives 132, 122 étant en regard l'une de l'autre (position basse du percuteur 130 (correspondant approximativement à la position représentée à la figure 11), le percuteur 130 est poussé progressivement vers le haut, contre l'action de l'organe élastique 109, jusqu'à accomplissement d'un tour complet (position haute du percuteur 130), puis est chassé contre le réducteur 120 par l'organe élastique 109 dont la libération brusque de l'énergie emmagasinée engendre une percussion transmise au dilatateur 1 solidaire du percuteur 130, ce dernier se retrouvant alors au point de départ précité, les méplats 133 et 123 étant à nouveau plaqués l'un contre l'autre. Le praticien répétera ces cycles de percussion, le dilatateur 1 s'enfonçant progressivement dans le lit implantaire tout en comprimant la matière osseuse, cela jusqu'à ce que le dilatateur 1 ait atteint la profondeur P définie du siège implantaire. Bien entendu, le praticien pourra moduler la pression axiale du dilatateur dans ledit siège comme il l'entend, en exerçant une force axiale (selon l'axe 1 A) dans une direction ou dans l'autre.

Pour accomplir la deuxième étape de compression de la matière osseuse (en nous référant plus particulièrement au premier des deux processus décrits plus haut à propos des types de mouvements imprimés au dilatateur), le praticien fait effectuer au dilatateur 1 une rotation - dont il décide l'ampleur angulaire - uniquement dans le sens horaire R1 ou alternativement dans le sens horaire R1 et anti-horaire R2, selon le type de dilatateur (voir supra) qu'il utilise. Pour ce faire, le praticien dégage l'extrémité 116 de l'arbre de sélection 115 hors de la fente 135 en tirant le bouton de sélection 117 vers l'arrière, ce qui procure au percuteur 130 une liberté en rotation. Simultanément, la coulisse 110, c'est-à-dire la protubérance 111 de celle-ci, s'engage dans la gorge 140 du percuteur 130 supprimant ainsi le degré de liberté du percuteur 130 en translation (ou degré de liberté du mouvement axial). Le percuteur 130, en position basse, est entraîné en rotation par le réducteur contre lequel l'organe élastique 109 le maintient en appui.

Lorsqu'au cours de la préparation osseuse le praticien estime qu'à un endroit donné il serait avantageux, par exemple pour effectuer momentanément ou sur un espace de temps plus long un travail de coupe (ou de raclage), ou pour vaincre une résistance due à une dureté - locale ou pouvant s'étendre sur une distance plus importante - de l'os, et dans l'hypothèse bien sûr que le dilatateur momentanément accroché au percuteur 130 soit du type à arête tranchante (voir variante 40 de la première forme de réalisation du dilatateur 1), le praticien fera tourner le dilatateur dans le sens anti-horaire R2, d'un angle qu'il détermine, qui sera souvent de quelques degrés seulement, mais qui pourrait être sensiblement plus grand et dépasser même les 360°. A cette fin, il inversera tout simplement le sens de rotation de R1 en R2 (voir alinéa précédent), sans se voir contraint de changer d'outil, c'est-à-dire sans devoir faire appel à un outil de coupe usuel.

L'exécution décrite comporte un système de sécurité, puisque si une rotation dans le sens R2 est commandée (inversion du sens de rotation de l'arbre de transmission 118), l'extrémité 116 de l'arbre de sélection 115 se dégage automatiquement de la fente 135, grâce à la forme arrondie ou évasée du flanc latéral gauche 136 de la fente 135 contre laquelle est placée ladite extrémité. De plus, dans ce sens anti-horaire, le face d'appui 122 du réducteur est poussée contre la face d'appui 132 du réducteur, empêchant tout glissement du percuteur, d'autant que le couple nécessaire à l'opération ponctuelle de coupe est plus grand que celui nécessaire à l'opération de compression osseuse.

L'homme du métier comprend à partir de cette forme d'exécution que les deux mouvements peuvent être combinés, le dilatateur étant alors soumis simultanément à un mouvement en translation et à un mouvement en rotation dans l'un quelconque des deux sens, conformément au second des deux processus décrits plus haut à propos des types de mouvements imprimés au dilatateur. Ainsi, à titre d'exemple, le dilatateur 1 peut être soumis, concomitamment au mouvement de translation, lui-même accompagné d'une percussion, à un mouvement en rotation dans un sens ou dans l'autre, d'angle limité, grâce à une conformation hélicoïdale des fentes 135, l'hélice pouvant être à pente droite ou gauche, au lieu d'être rectilignes.

Ainsi, le dispositif 100 présente un avantage supplémentaire de grand intérêt, à savoir celui de la polyvalence: au-delà de l'application privilégiée de préparation de siège implantaire dont on vient de parler, ce dispositif permet en effet d'exécuter toutes fonctions traditionnelles de forage.

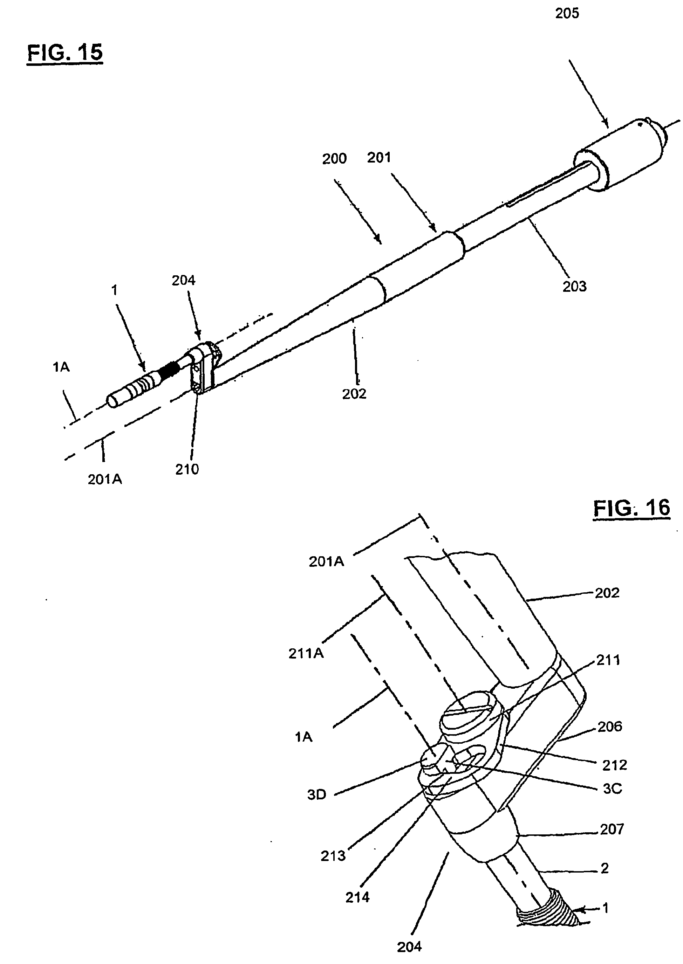

A titre d'illustration, deux formes de réalisation de dispositifs moins sophistiquées, entrant dans la deuxième catégorie invoquée (dispositifs manuels), vont à présent être décrites à l'appui des figures 15 à 19 (premier exemple de forme de réalisation) et 20 à 22 (deuxième exemple de forme de réalisation).

Le dispositif 200 (figure 15) comporte un manche 201 d'axe 201 A et composé d'une partie avant 202 et d'une partie arrière 203, une tête de fixation 204 d'un dilatateur 1 d'axe 1 A agencée à l'extrémité avant de la partie 202 du manche 201, et un dispositif de percussion 205 agencé dans la zone arrière de la partie 203 du manche 201 et coopérant avec ladite partie 203.

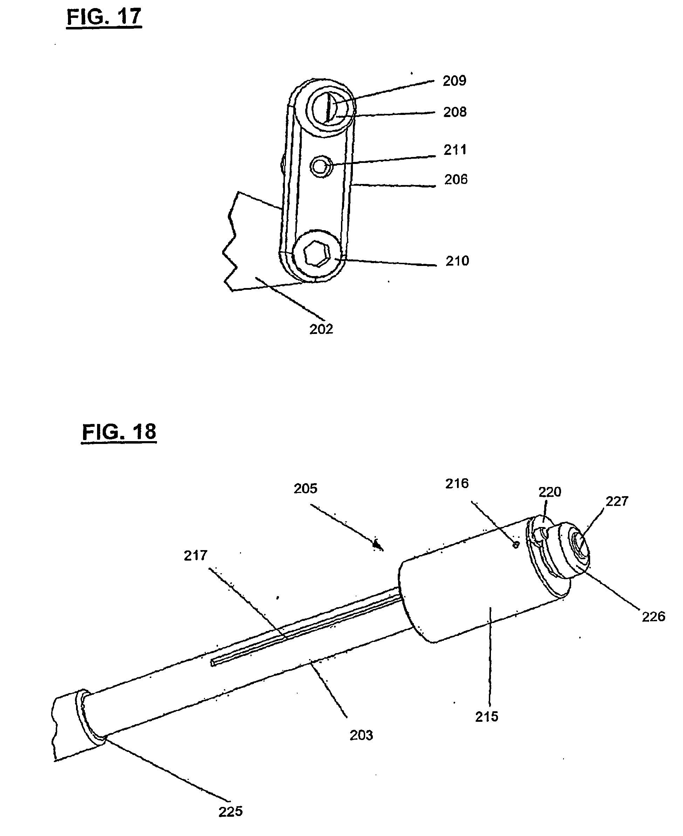

La tête 204 (voir figures 16 et 17) est une pièce de forme 206 présentant à son extrémité avant un talon 207 et une ouverture 208 d'axe 1 A dans laquelle la tige 2 du dilatateur 1 (figure 1) peut être engagée ou en être dégagée, la partie supérieure de cette ouverture 208 étant partiellement obturée par une butée 209. L'extrémité arrière de la pièce 206 présente, dans le prolongement de l'axe 201 A, une ouverture (non référencée) dans laquelle prend place un organe de fixation 210 reliant la tête 204 au manche 201. Entre les deux ouvertures précitées est aménagée une troisième ouverture (non référencée) dans laquelle est monté un tourillon 211 d'axe 211 A autour duquel peut pivoter un verrou 212 agencé sur la face supérieure (non référencée) de la pièce 206 et se présentant sous forme de plaquette approximativement triangulaire aux angles très arrondis. Le verrou comporte à l'avant de l'axe de pivotement 211 A deux ouvertures 213, 214 (d'axes non référencés parallèles à l'axe 1 A) mordant l'une dans l'autre, le diamètre de l'ouverture 213 correspondant au diamètre 3 B de la gorge de tige du dilatateur 1 (pour les références 3 A, 3 B, 3 C, 3 D, voir figure 1), le diamètre de l'ouverture 214 correspondant au diamètre du chapeau 3 D. Le verrou 212 permet de lier le dilatateur 1 à la tête 204 en translation lorsqu'il est rabattu dans la gorge 3 B du dilatateur, après introduction de celui-ci jusqu'à ce que l'épaulement 3 A vienne contre la butée 209, le méplat 3 C faisant face à ladite butée assurant la liaison en rotation du dilatateur (position montrée à la figure 16).

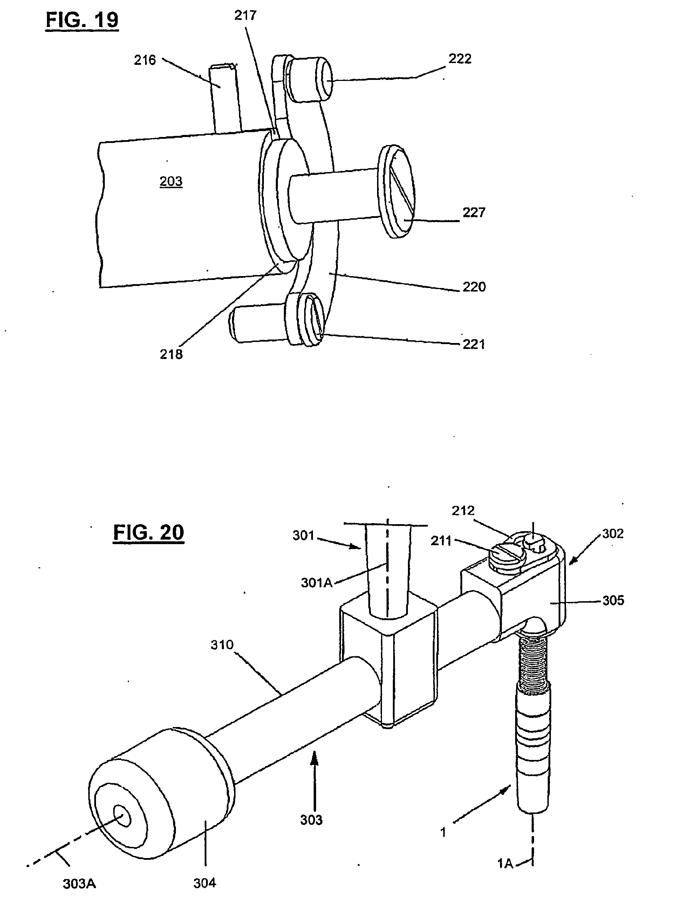

Le dispositif de percussion 205 (figures 18 et 19) comprend une masse 215 pouvant coulisser le long de la partie 203 de l'arbre 201, tout en étant de préférence liée en rotation grâce à un élément 216 dont l'extrémité (non représentée) est guidée dans une rainure 217 de la partie 203. La masse peut être immobilisée à l'extrémité de la partie 203 grâce à un loquet 220 agencé sur sa face supérieure (non référencée), ce loquet étant pivotant autour d'un tourillon 221, par commande à la main d'un tenon 222 et pouvant être rabattu contre une gorge 218 prévue dans la partie supérieure de l'élément 203 du manche 201 (figure 19, loquet 220 à l'état rabattu). Le dispositif de percussion comprend enfin un bouton 226 fixé sur la face supérieure (non référencée) de la partie 203 du manche 201 par un organe de fixation 227.

Pour effectuer l'insertion axiale du dilatateur dans le siège implantaire, le praticien libère la masse 215 en ouvrant le loquet 220 et la frappe contre un épaulement 225 (figure 18) prévu à la partie supérieure de la partie 202 du manche 201). Lorsque le dilatateur est arrivé à la profondeur voulue, le praticien procède à la compression osseuse en faisant tourner le dispositif 200 à la main. Bien entendu, il est également possible d'imprimer au dilatateur un mouvement de rotation simultanément à la percussion. Pour extraire le dilatateur du siège implantaire, le praticien peut, le cas échéant, frapper la masse 215 contre le bouton 226.

Le dispositif peut être réalisé avec des manches 201 de longueurs différentes (par exemple un jeu de parties de manche 202 de différentes longueur, ou en prévoyant un élément 202 télescopique), pour assurer une bonne accessibilité en toutes circonstances. D'autre part, il est évident qu'une tête de fixation pourrait être construite de sorte que l'axe 1 A du dilatateur 1 et l'axe 201 A du manche 201 aient un même support.

Le dispositif 300 représenté aux figures 20, 21 et 22 se compose d'un manche 301 d'axe 301 A (quasiment identique au manche 201 tel que décrit plus haut) fixé à un bras 303, d'axe 303 A, à l'une des extrémités duquel est monté une tête de contre-angle 302 (connue en soi) retenant le dilatateur 1 d'axe 1 A, l'autre extrémité comportant une molette ou roulette 304. Selon l'exemple, les axes 1 A, 301 A et 303 A sont dans un même plan et les axes 1 A et 301 A sont parallèles entre eux.

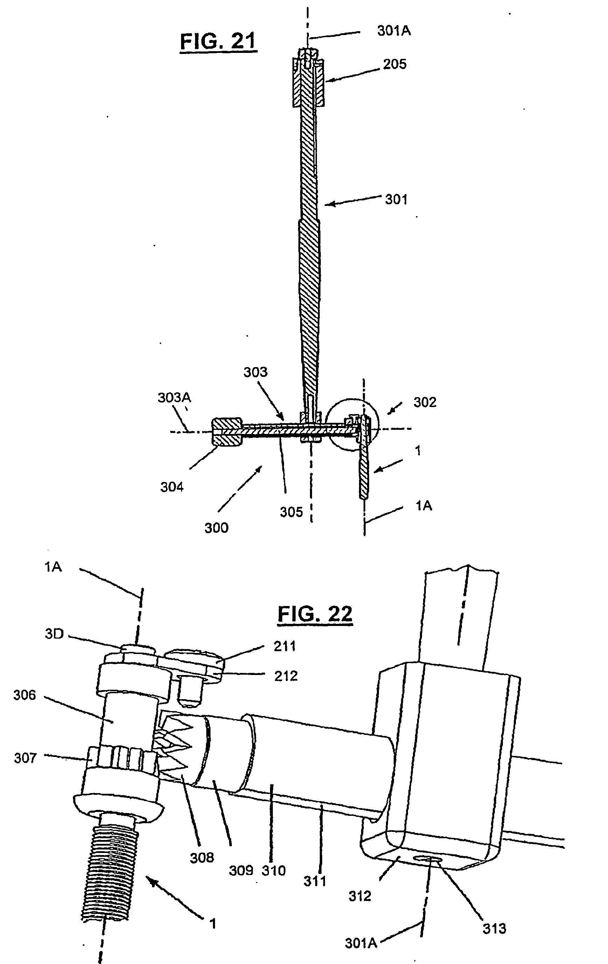

Le dilatateur 1 est lié en translation par un même système de verrouillage 211, 212 que celui qui a été décrit à propos du dispositif 200. Mais à la différence de ce dernier, le dilatateur peut être soumis à un entraînement en rotation grâce à un mécanisme logé dans un carter 305 de la tête 302 (voir figure 22 où ce mécanisme est visible, la tête 302 étant représentée sans le carter). Ce mécanisme comprend un manchon d'entraînement 306 à l'intérieur duquel une butée coopère avec l'épaulement 3 A et le méplat 3 C du dilatateur (ladite butée, l'épaulement 3 A et le méplat 3 C n'étant pas visibles sur les figures, étant observé que l'homme du métier, connaissant ce mécanisme, peut envisager tous autres éléments de construction pour assurer la liaison dilatateur - manchon). Le manchon 306 est pourvu d'une couronne dentée 307 en prise avec un pignon à denture frontale 308 constituant la partie terminale d'un arbre 309 guidé dans un tube 310 du bras 303. La molette 304 est fixée à l'extrémité opposée de l'arbre 309 par un organe de fixation non représenté.

Une noix 312 peut coulisser le long du tube 310 et être immobilisée sur ce dernier à une position définie au moyen d'une vis de pression 313, eu égard aux conditions d'accessibilité du lit implantaire dans la bouche du patient. Afin de préserver une rotation intempestive de la noix 312, et plus particulièrement le parallélisme entre l'axe 301 A et l'axe 1 A du dilatateur, le tube 310 du bras 303 comporte un méplat 311 contre lequel la vis de pression vient en appui. Le manche 301 est fixé sur la noix sur la face supérieure (non référencée) de celle-ci, par exemple par vissage.

Après exercice du mouvement de translation par actionnement du dispositif de percussion 205 (cf. figure 21 et explications données plus haut à propos du dispositif 200), le mouvement de rotation du dilatateur - étant précisé que le dispositif 300 permet une rotation dans le sens horaire et anti-horaire (actions de compression et de raclage ou de coupe) - est commandé par une action sur la roulette 304 (dans la plupart des cas, le nombre de tours que le praticien fera faire au dilatateur est au plus égal à deux, et sera souvent inférieur à un). Tout comme avec le dispositif 200, le dilatateur peut, tout en étant soumis à une translation le long de son axe 1 A, être actionné en rotation autour de ce même axe.

Il va de soi que nombre d'autres formes de réalisation ou variantes de dispositifs manuels ou automatiques peuvent être conçus, sans sortir du cadre de la présente invention. On observera dans ce contexte qu'au-delà de l'aspect rationnel procuré par la possibilité de détacher le dilatateur, quel qu'il soit, du dispositif mis en oeuvre, le fait de pourvoir les manches des dispositifs manuels d'un système d'actionnement en translation, avantageusement par percuteur comportant une masse, de même que la création automatique de frappes axiales (dispositif automatique), moyens inexistants à ce jour, permet notamment d'améliorer la maîtrise du calibrage d'un siège implantaire, cela même en utilisant les dilatateurs conventionnels cylindriques (pointeaux), a fortiori lorsqu'il est fait usage des dilatateurs décrits plus haut et revendiqués. De plus, l'application de ces dispositifs peut s'étendre au-delà des seules préparations de sièges osseux. En particulier, ils s'avèrent parfaitement adaptés pour recevoir des outils aux fins d'effectuer des interventions de surélévation du sinus maxillaire (par la technique de surélévation d'un fragment osseux alvéolaire).

Les outils de préparation osseuse et leurs dispositifs de mise en oeuvre selon l'invention constituent les deux piliers d'un système intégré hautement innovant - à juste titre dénommé "SDD" ("système de dilatation en douceur") ou "SDS" ("soft dilating system") - et apportant sa pierre à l'édifice en étendant considérablement les domaines d'applications de l'implantologie.

## Revendications

1. Outil de compression osseuse utilisable notamment en médecine dentaire pour préparer un siège osseux en vue de la pose d'un implant à insertion axiale, notamment pour remplacer une dent naturelle, cet outil comportant une partie active permettant dé façonner ledit siège osseux et pouvant se terminer par une partie apicale,
**caractérisé en ce que** la partie actives présente une section droite dont là forme est définie de telle sorte qu'un mouvement en rotation de l'outil autour d'un axe, a pour effet de comprimer la matière osseuse vers la périphérie de ce siège et d'imprimer simultanément à ce dernier une forme déterminée induite des paramètres anatomiques du siège osseux de la dent naturelle, son ampleur angulaire étant essentiellement fonction chaque fois du type de countour de la section de l'outil et des qualités mécaniques de la matière osseuse travaillée, et qu'elle peut être orientée dans le sens horaire ou anti-horaire, ou être alternative dans un sens est dans le sens inverse.

2. Outil selon la revendication 1, **caractérisé en ce que** la rotation est précédée ou accompagnée d'un mouvement de translation dans le sens dudit axe dans le siège osseux.

3. Outil selon la revendication 1 ou 2, **caractérisé en ce que** la forme de ladite section droite est autre que circulaire, le caractère non circulaire étant défini par le fait que l'écart relatif entre le diamètre du cercle passant par le point ou l'ensemble de points de la section droite le plus éloigné de l'axe de rotation et le diamètre-du cercle passant par le point ou l'ensemble de points de la section le plus rapproché dudit axe est égal ou supérieur à 0.5 %.

4. Outil selon l'une des revendications 1 à 3, **caractérisé en ce que** ladite section droite est au moins approximativement de forme polygonale, les sommets du polygone présentant de préférence une forme courbe, ou dé segment circulaire, ou de méplat.

5. Outil selon la revendication 4, **caractérisé en ce que** le polygone est de préférence du type pentagone, hexagone ou heptagone.

6. Outil selon la revendication 4 ou 5, **caractérisé en ce que** la forme polygonale est modifiée, au moins un des côtés pouvant être incurvé vers l'axe de rotation ou présenter tout autre tracé courbe, notamment sinusoïdal, ou combiné droit/courbe.

7. Outil selon l'une des revendications 1 à 3, **caractérisé en ce que** la forme de ladite section droite est au moins approximativement en astéroïde ou pétaloïde à au moins trois branches.

8. Outil selon l'une des revendications 1 à 3, **caractérisé en ce que** ladite section droite présente au moins une partie excentrée.

9. Outil selon l'une des revendications 1 à 3, 7 ou 8, **caractérisé en ce que** la forme de ladite section droite est approximativement en ovoïde avec un sommet de préférence arrondi ou en forme de segment circulaire.

10. Outil selon l'une des revendications 1 à 3, 7 ou 8, **caractérisé en ce que** la forme de ladite section droite est approximativement en double ovoïde avec deux sommets opposés de préférence en forme d'arrondi ou de segment circulaire.

11. Outil selon l'une des revendications 1 à 10, **caractérisé en ce que** la partie active présente, dans un plan axial, un profil déterminé en fonction du profil axial souhaité du siège osseux.

12. Outil selon la, revendication 11, **caractérisé en ce que** le profil de la parties active peut être droit, courbe ou combiné droit/courbe.

13. Outil selon la revendication 11 ou 12, **caractérisé en ce que** le profil de la partie active présente une forme étagée par succession d'épaulements de dimensions allant décroissantes en direction de la partie apicale.

14. Outil selon l'une des revendications 1 à 13, **caractérisé en ce que** la partie active comporte au moins une arête et que cette arête est droite ou hélicoïdale.

15. Outil selon la revendication 14, **caractérisé en ce que** au moins une arête est vive, arrondie, ou en forme de méplat.

16. Outil selon l'une des revendications 1 à 15, **caractérisé en ce que** la partie active présente deux sous-parties, une sous-partie arrière étant prolongée d'une sous-partie avant conique aboutissant à la partie apicale, la conicité étant avantageusement de l'ordre de 1°

17. outil selon la revendication 16, **caractérisé en ce que** la longueur de là sous-partie avant correspond au moins approximativement à la profondeur du siège implantaire.

18. Outil selon l'une des revendications 14 à 17, **caractérisé en ce que** sa rotation dans un sens provoque une compression de la paroi osseuse du siège implantaire et que sa rotation dans le sens inverse provoque une coupe ou un raclage de la paroi osseuse du siège implantaire, au moins une arête étant tranchante.

19. outil selon l'une des revendications 11 à 18, **caractérisé en ce que** la partie apicale peut exercer une action de coupe, soit lors de l'un seulement des deux sens de rotation, soit lors des deux sens de rotation.

20. Dispositif de mise en oeuvre muni d'un outil de compression osseuse selon l'une des revendications 1 à 19, comportant des moyens permettant de transmette audit outil un mouvement en translation dans le sens de l'axe dudit outil et un mouvement en rotation.

21. Dispositif selon la revendication 20, **caractérisé en ce que** le moyen transférant le mouvement en translation est formé d'un système de percussion comportant un percuteur coopérant avec un organe de transmission d'un mouvement de rotation, l'outil étant relié au percuteur.

22. Dispositif selon la revendication 21, **caractérisé en ce que** des moyens de coopération entre le percuteur et l'organe de transmission permettent de soumettre le percuteur à un mouvement de va-et-vient d'amplitude-définie, et audit mouvement de rotation ou à une combinaison de ces mouvements, le mouvement résultant étant transmis à l'outil.

23. Dispositif selon la revendication 22, **caractérisé en ce que** les moyens de coopération sont formés de deux rampes complémentaires se faisant face et disposées, l'une sur l'organe de transmission du mouvement en rotation, l'autre sur le percuteur, ce dernier étant soumis à l'action d'un organe élastique.

24. Dispositif selon la revendication 22 ou 23, **caractérisé en ce qu'**il comporte des moyens de sélection de sorte que l'outil effectue le mouvement ou la combinaison de mouvements voulue.

25. Dispositif selon la revendication 24, **caractérisé en ce que** les moyens de sélection sont formés d'un bras de sélection dont l'extrémité coopère avec au moins une ouverture que comporte le percuteur, le bras de sélection étant commandé au moyen d'un mécanisme de sélection, de sorte à imposer au percuteur le mouvement en translation combiné ou non avec le mouvement en rotation.

26. Dispositif selon la revendication 25, **caractérisé en ce que** l'ouverture présente un flanc arrondi permettant due provoquer une libération automatique de l'extrémité du bras de sélection hors de ladite ouverture lorsque le percuteur est soumis à une rotation dans un sens défini.

27. Dispositif selon la revendication 20, **caractérisé en ce que** le système de percussion est disposé sur une, partie d'un manche comportant un épaulement, l'outil étant engagé dans une tête à l'extrémité opposée du manche et lié en rotation et en translation.

28. Dispositif selon la revendication 27, **caractérisé en ce que** la liaison en translation est assurée par un verrou pouvant pivoter autour d'un tourillon.

29. Dispositif selon la revendication 27 ou 28, **caractérisé en ce que** le système de percussion comprend une masse pouvant coulisser sur une distance, le long d'une partie du manche et frapper contre un épaulement prévu sur le manche.

30. Dispositif selon la reoendication 29, **caractérisé en ce que** la masse est liée en rotation.

31. Dispositif selon la revendication 29 ou 30, **caractérisé en ce que** la partie extrême du manche comporte un arrêt contre lequel la masse peut venir frapper afin de faciliter l'extraction de l'outil d'un pertuis.

32. Dispositif selon l'une des revendications 29 à 31, **caractérisé en ce que** la masse peut, être bloquée à une extrémité du manche au moyen, d'un, mécanisme, de verrouillage.

33. Dispositif selon, les revendications 27 à 32, **caractérisé en ce qu'**il peut être soumis manuellement à un mouvement en rotation autour de l'axe de l'outil, de sorte à actionner ce dernier en rotation.

34. Dispositif selon la revendication 20 **caractérisé en ce que** le système de percussion est disposé sur une partie d'un manche comportant un épaulement, ce manche étant rélié à un bras à l'une des extrémités duquel est agencée une tête recevant l'outil dont l'axe s'étend orthogonalement à l'axe dudit bras et parallèlement à l'axe du manche.

35. Dispositif selon la revendication 34 **caractérisé en ce que** le système de percussion correspond à celui défini dans l'une ou l'autre des revendications 29 à 32.

36. Dispositif selon la revendication 35 **caractérisé en ce que** le manche pourvu de son système de percussion est relié au bras par un élément de ponction pouvant coulisser le long du bras et être immobilisé sur celui-ci à l'aide d'un moyen de blocage.

37. Dispositif selon la revendication 36 **caractérisé en ce que** l'élément de jonction est lié en rotation sur le bras, de sorte à maintenir le parallélisme de l'axe de l'outil et de l'axe du manche.

38. Dispositif selon la revendication 37 **caractérisé en ce que** la liaison en rotation est assurée par un méplat que présente le bras, l'élément de jonction étant pourvu d'un méplat correspondant.

39. Dispositif selon l'une des revendications 34 à 38, **caractérisé en ce que** l'outil introduit dans la tête est lié en translation au moyen d'un verrou et est libre en rotation, l'outil étant lié à un manchon rotatif.

40. Dispositif selon la revendication 39, **caractérisé en ce que** le manchon est en prise avec un arbre de transmission pouvant être actionné au moyen d'une roulette, de sorte à permette l'actionnement en rotation de l'outil.

## Claims

1. Osseous compression tool for use particularly in dentistry for the preparation of an osseous seat in view of the placement of an axially inserted implant, particularly for replacing a natural tooth, the tool comprising an active portion that allows shaping said osseous seat and may be terminated by an apical portion,
**characterised in that** the cross-sectional shape of the active portion is defined such that a rotational movement of the tool around an axis has the effect that the osseous matter is compressed towards the periphery of the seat and that the latter is simultaneously imparted a determined shape that is induced by the anatomical parameters of the osseous seat of the natural tooth, while its angular width is essentially determined by the respective type of contour of the cross-section of the tool and by the mechanical qualities of the worked osseous matter, and **in that** it may be oriented in the clockwise or in the counterclockwise direction or reciprocating in one and in the opposite direction.

2. Tool according to claim 1, **characterised in that** the rotation is preceded or accompanied by a translational movement in the direction of said axis inside the osseous seat.

3. Tool according to claim 1 or 2, **characterised in that** said cross-sectional shape is other than circular, the non-circular character being defined as the fact that the relative distance between the diameter of the circle passing through the point or the set of points of the cross-section furthest from the rotation axis and the diameter of the circle passing through the point or the set of points of the cross-section nearest to said axis is equal to or greater than 0.5 %.

4. Tool according to one of claims 1 to 3, **characterised in that** said cross-section is at least approximately polygonal in shape, the apexes of the polygon preferably having a curved shape or that of a circle segment or of a flat.

5. Tool according to claim 4, **characterised in that** the polygon is preferably of the pentagon, hexagon or heptagon type.

6. Tool according to claim 4 or 5, **characterised in that** the polygonal shape is modified, at least one of the sides being incurved towards the rotation axis or corresponding to any other curved contour, more particularly a sinusoidal one or a combination of straight and curved portions.

7. Tool according to one of claims 1 to 3, **characterised in that** said cross-sectional shape is at least approximately asteroid or petaloid with at least three branches.

8. Tool according to one of claims 1 to 3, **characterised in that** said cross-section comprises at least one eccentric portion.

9. Tool according to one of claims 1 to 3, 7 or 8,
**characterised in that** said cross-sectional shape is approximately ovoid with an apex that is preferably rounded or in the shape of a circle segment.

10. Tool according to one of claims 1 to 3, 7 or 8,
**characterised in that** said cross-sectional shape is approximately that of a double ovoid with two opposite apexes that are preferably rounded or in the shape of circle segments.

11. Tool according to one of claims 1 to 10, **characterised in that** in an axial plane, the active portion has a profile that is determined in function of the intended axial profile of the osseous site.

12. Tool according to claim 11, **characterised in that** the profile of the active portion may be straight, curved or a combination of straight and curved.

13. Tool according to claim 11 or 12, **characterised in that** the profile of the active portion is stepped in a succession of shoulders whose dimensions decrease towards the apical portion.

14. Tool according to one of claims 1 to 13, **characterised in that** the active portion comprises at least one edge and **in that** this edge is straight or helical.

15. Tool according to claim 14, **characterised in that** at least one edge is sharp, rounded, or in the shape of a flat.

16. Tool according to one of claims 1 to 15, **characterised in that** the active portion comprises two subsections, a rear subsection being followed by a conical front subsection ending in the apical portion, the conicity preferably being of the order of 1°.

17. Tool according to claim 16, **characterised in that** the length of the front subsection corresponds to the depth of the implant seat at least approximately.

18. Tool according to one of claims 14 to 17,
**characterised in that** its rotation in one direction causes a condensation of the osseous wall of the implant seat and its rotation in the opposite direction causes a cutting or scraping of the osseous wall of the implant seat, at least one edge being a cutting edge.

19. Tool according to one of claims 11 to 18,
**characterised in that** the apical portion may exert a cutting action, either in only one of the two rotational directions or in both rotational directions.

20. Operating device provided with an osseous condensation tool according to one of claims 1 to 19, comprising means for imparting the tool a translational movement in the direction of the axis of said tool as well as a rotational movement.

21. Device according to claim 20, **characterised in that** the means transmitting the translational movement is formed of a percussion system comprising a striker that cooperates with a member for transmitting a rotational movement, the tool being connected to the striker.

22. Device according to claim 21, **characterised in that** means for the cooperation between the striker and the transmission member allow to subject the striker to a reciprocating movement of a defined amplitude and to said rotational movement or to a combination of such movements, the resulting movement being transmitted to the tool.

23. Device according to claim 22, **characterised in that** the cooperation means are formed of two complementary ramps which face each other and are disposed on the member for the transmission of the rotational movement and on the striker, respectively, the latter being subject to the action of an elastic member.

24. Device according to claim 22 or 23, **characterised in that** it comprises selecting means such that the tool performs the intended movement or combination of movements.

25. Device according to claim 24, **characterised in that** the selecting means are formed of a selector arm whose end cooperates with at least one opening of the striker, the selector arm being controlled by means of a selecting mechanism in such a manner as to impart the striker the translational movement combined or not combined with the rotational movement.

26. Device according to claim 25, **characterised in that** the opening comprises a rounded flank that allows an automatic liberation of the end of the selector arm from said opening when the striker is rotated in a given direction.

27. Device according to claim 20, **characterised in that** the percussion system is disposed on a portion of a handle comprising a shoulder, the tool being engaged in a head at the opposite end of the handle and rotationally and translationally locked.

28. Device according to claim 27, **characterised in that** the translational lock is ensured by a latch that may pivot around a pivot.

29. Device according to claim 27 or 28, **characterised in that** the percussion system comprises a weight that may slide over a distance along a portion of the handle and strike a shoulder provided on the handle.

30. Device according to claim 29, **characterised in that** the weight is rotationally locked.

31. Device according to claim 29 or 30, **characterised in that** the end portion of the handle comprises a stop that the weight may strike in order to facilitate the extraction of the tool from a hole.

32. Device according to one of claims 29 to 31,
**characterised in that** the weight can be locked to one end of the handle by means of a locking mechanism.

33. Device according to claims 27 to 32, **characterised in that** it is manually rotatable around the axis of the tool, thereby actuating the latter rotationally.

34. Device according to claim 20, **characterised in that** the percussion system is disposed on a portion of a handle comprising a shoulder, the handle being connected to an arm one end of which is provided with a head for receiving the tool whose axis extends perpendicularly to the axis of said arm and in parallel to the axis of the handle.

35. Device according to claim 34, **characterised in that** the percussion system corresponds to that defined in any one of claims 29 to 32.

36. Device according to claim 35, **characterised in that** the handle provided with its percussion system is connected to the arm by a connecting element that is slidable along the arm and may be locked thereon by means of a locking means.

37. Device according to claim 36, **characterised in that** the connecting element is rotationally locked on the arm such as to maintain the parallelism between the tool axis and the axis of the handle.

38. Device according to claim 37, **characterised in that** the rotational lock is ensured by a flat provided on the arm, the connecting element being provided with a corresponding flat.

39. Device according to one of claims 34 to 38,
**characterised in that** the tool inserted in the head is translationally locked by means of a catch and is freely rotatable, the tool being fastened to a rotatable sleeve.

40. Device according to claim 39, **characterised in that** the sleeve is in engagement with a transmission shaft which is actuatable by means of a wheel, thereby allowing the rotational actuation of the tool.

## Patentansprüche

1. Knochenkompressionswerkzeug, verwendbar insbesondere in der Zahnmedizin zur Vorbereitung eines Knochenlagers für den Einsatz eines axial einzuführenden Implantats, insbesondere für den Ersatz eines natürlichen Zahns, wobei das Werkzeug einen aktiven Teil zum Formen des Knochenlagers aufweist und am Ende in einen apikalen Teil übergehen kann, **dadurch gekennzeichnet, dass** der aktive Teil einen Querschnitt aufweist, dessen Form derart festgelegt ist, dass eine Drehbewegung des Werkzeugs um eine Achse eine Kompression des Knochenmaterials zur Peripherie dieses Lagers und gleichzeitig eine bestimmte Formgebung desselben bewirkt, die von den anatomischen Parametern des Knochenlagers des natürlichen Zahns abgeleitet wird, wobei ihr Winkelbereich jeweils im Wesentlichen von der Art der Konturen des Werkzeugquerschnitts und von den mechanischen Eigenschaften des bearbeiteten Knochenmaterials abhängt, und dass sie im Uhrzeiger- oder im Gegenuhrzeigersinn erfolgen kann oder abwechselnd in einer Richtung und in der Gegenrichtung.

2. Werkzeug nach Anspruch 1, **dadurch gekennzeichnet, dass** vor oder während der Drehung eine translatorische Bewegung in der Richtung der genannten Achse im Knochenlager erfolgt.

3. Werkzeug nach Anspruch 1 oder 2, **dadurch gekennzeichnet, dass** die Form des genannten Querschnitts nicht rund ist, wobei die Unrundheit dadurch definiert ist, dass der relative Abstand zwischen dem Durchmesser des Kreises, der durch den bzw. die am weitesten von der Drehachse entfernten Punkt oder Punktemenge des Querschnitts verläuft, und dem Durchmesser des Kreises, der durch den bzw. die am nächsten bei der Drehachse liegenden Punkt oder Punktemenge des Querschnitts verläuft, gleich oder grösser als 0,5 % ist.

4. Werkzeug nach einem der Ansprüche 1 bis 3, **dadurch gekennzeichnet, dass** der genannte Querschnitt mindestens annähernd polygonförmig ist, wobei die Ecken des Polygons vorzugsweise gekrümmt, kreissegmentförmig oder abgeflacht sind.

5. Werkzeug nach Anspruch 4, **dadurch gekennzeichnet, dass** das Polygon vorzugsweise ein Pentagon, Hexagon oder Heptagon ist.

6. Werkzeug nach Anspruch 4 oder 5, **dadurch gekennzeichnet, dass** die Polygonform abgewandelt ist, wobei mindestens eine Seite zur Drehachse gekrümmt sein oder beliebig anders gekrümmt verlaufen kann, insbesondere sinusförmig oder kombiniert gerade/gekrümmt.

7. Werkzeug nach einem der Ansprüche 1 bis 3, **dadurch gekennzeichnet, dass** der genannte Querschnitt mindestens annähernd stern- oder blattförmig mit mindestens drei Schenkeln ist.

8. Werkzeug nach einem der Ansprüche 1 bis 3, **dadurch gekennzeichnet, dass** der genannte Querschnitt mindestens einen exzentrischen Teil aufweist.

9. Werkzeug nach einem der Ansprüche 1 bis 3, 7 oder 8, **dadurch gekennzeichnet, dass** der genannte Querschnitt annähernd ovoid mit einem vorzugsweise abgerundeten oder kreissegmentförmigen Scheitel ist.

10. Werkzeug nach einem der Ansprüche 1 bis 3, 7 oder 8, **dadurch gekennzeichnet, dass** der genannte Querschnitt annähernd doppelt ovoid mit zwei gegenüberliegenden, vorzugsweise abgerundeten oder kreissegmentförmigen Scheiteln ist.

11. Werkzeug nach einem der Ansprüche 1 bis 10, **dadurch gekennzeichnet, dass** der aktive Teil in einer Axialebene ein entsprechend dem gewünschten axialen Profil des Knochenlagers festgelegtes Profil aufweist.

12. Werkzeug nach Anspruch 11, **dadurch gekennzeichnet, dass** das Profil des aktiven Teils gerade, gekrümmt oder kombiniert gerade/gekrümmt sein kann.

13. Werkzeug nach Anspruch 11 oder 12, **dadurch gekennzeichnet, dass** das Profil des aktiven Teils eine abgestufte Form durch eine Reihe von Absätzen aufweist, deren Abmessungen gegen den apikalen Teil abnehmen.

14. Werkzeug nach einem der Ansprüche 1 bis 13, **dadurch gekennzeichnet, dass** der aktive Teil mindestens eine Kante aufweist und diese Kante gerade oder schraubenförmig ist.

15. Werkzeug nach Anspruch 14, **dadurch gekennzeichnet, dass** mindestens eine Kante scharf, abgerundet oder abgeflacht ist.

16. Werkzeug nach einem der Ansprüche 1 bis 15, **dadurch gekennzeichnet, dass** der aktive Teil zwei Unterpartien aufweist, wobei eine hintere Unterpartie in eine vordere Unterpartie übergeht, welche im apikalen Teil endet, wobei die Konizität vorteilhaft in der Grössenordnung von 1° liegt.

17. Werkzeug nach Anspruch 16, **dadurch gekennzeichnet, dass** die Länge der vorderen Unterpartie mindestens annähernd der Tiefe des Implantatlagers entspricht.

18. Werkzeug nach einem der Ansprüche 14 bis 17, **dadurch gekennzeichnet, dass** dessen Drehung in einer Richtung eine Kompression der Knochenwand des Implantatlagers bewirkt und dessen Drehung in der Gegenrichtung ein Schneiden bzw. Abschaben der Knochenwand des Implantatlagers, wobei mindestens eine Kante schneidet.

19. Werkzeug nach einem der Ansprüche 11 bis 18, **dadurch gekennzeichnet, dass** der apikale Teil eine Schneidwirkung ausüben kann, entweder in nur einer der beiden Drehrichtungen oder in beiden Drehrichtungen.

20. Inbetriebvorrichtung mit einem Knochenkompressionswerkzeug nach einem der Ansprüche 1 bis 19, die Mittel enthält, die es gestatten, eine translatorische Bewegung in der Richtung der Werkzeugachse und eine Drehbewegung auf das genannte Werkzeug zu übertragen.

21. Vorrichtung nach Anspruch 20, **dadurch gekennzeichnet, dass** das Mittel zur Übertragung der translatorischen Bewegung aus einer Schlagvorrichtung mit einem Schlagbolzen besteht, das mit einem Organ zur Übertragung einer Drehbewegung zusammenwirkt, wobei das Werkzeug mit dem Schlagbolzen verbunden ist.

22. Vorrichtung nach Anspruch 21, **dadurch gekennzeichnet, dass** Mittel für das Zusammenwirken des Schlagbolzens mit dem Übertragungsorgan den Schlagbolzen mit einer Hin- und Herbewegung einer festgelegten Amplitude und mit der genannten Drehbewegung oder mit einer Kombination dieser Bewegungen zu beaufschlagen gestatten, wobei die resultierende Bewegung an das Werkzeug übertragen wird.

23. Vorrichtung nach Anspruch 22, **dadurch gekennzeichnet, dass** die Mittel für das Zusammenwirken aus zwei komplementären, einander gegenüberstehenden Rampen bestehen, von welchen die eine auf dem Übertragungsorgan für die Drehbewegung und die andere auf dem Schlagbolzen angeordnet ist, wobei auf letzteres ein Federorgan wirkt.

24. Vorrichtung nach Anspruch 22 oder 23, **dadurch gekennzeichnet, dass** sie Wählmittel aufweist, so dass das Werkzeug die gewünschte Bewegung oder Bewegungskombination ausführt.

25. Vorrichtung nach Anspruch 24, **dadurch gekennzeichnet, dass** die Wählmittel aus einem Wählarm bestehen, dessen Ende mit mindestens einer Öffnung des Schlagbolzens zusammenwirkt, wobei der Wählarm mittels eines Wählmechanismus betätigt wird, um die translatorische Bewegung mit der Drehbewegung kombiniert oder ohne dieselbe an den Schlagbolzen zu übertragen.

26. Vorrichtung nach Anspruch 25, **dadurch gekennzeichnet, dass** die Öffnung eine abgerundete Flanke aufweist, welche es gestattet, eine automatische Freigabe des Wählarmendes aus der genannten Öffnung herbeizuführen, wenn eine Drehung des Schlagbolzens in einer bestimmten Richtung erfolgt.

27. Vorrichtung nach Anspruch 20, **dadurch gekennzeichnet, dass** die Schlagvorrichtung auf einem abgesetzten Teil eines Schafts angeordnet ist, wobei das Werkzeug in einem Kopf am entgegengesetzten Ende des Schafts eingespannt und drehend und translatorisch mit demselben verbunden ist.

28. Vorrichtung nach Anspruch 27, **dadurch gekennzeichnet, dass** die translatorische Verbindung durch einen Riegel erfolgt, der um einen Zapfen schwenkbar ist.

29. Vorrichtung nach Anspruch 27 oder 28, **dadurch gekennzeichnet, dass** die Schlagvorrichtung eine Masse beinhaltet, die auf einem Teil des Schafts über eine Strecke verschiebbar ist und auf einen auf dem Schaft vorgesehenen Absatz schlägt.

30. Vorrichtung nach Anspruch 29, **dadurch gekennzeichnet, dass** die Masse unverdrehbar ist.

31. Vorrichtung nach Anspruch 29 oder 30, **dadurch gekennzeichnet, dass** der Endteil des Schafts einen Anschlag aufweist, gegen den die Masse geschlagen werden kann, um das Herausziehen des Werkzeugs aus einer Bohrung zu erleichtern.

32. Vorrichtung nach einem der Ansprüche 29 bis 31, **dadurch gekennzeichnet, dass** die Masse an einem Ende des Schafts mittels eines Verriegelungsmechanismus arretierbar ist.

33. Vorrichtung nach den Ansprüchen 27 bis 32, **dadurch gekennzeichnet, dass** sie manuell um die Werkzeugachse drehbar ist, um dieses in Drehung zu versetzen.

34. Vorrichtung nach Anspruch 20, **dadurch gekennzeichnet, dass** die Schlagvorrichtung auf einem abgesetzten Teil eines Schafts angeordnet ist, der mit einem Arm verbunden ist, an einem Ende dessen ein Kopf angeordnet ist, der das Werkzeug aufnimmt, dessen Achse senkrecht zur Achse des Arms und parallel zur Achse des Schafts steht.

35. Vorrichtung nach Anspruch 34, **dadurch gekennzeichnet, dass** die Schlagvorrichtung jener gemäss einem der Ansprüche 29 bis 32 entspricht.

36. Vorrichtung nach Anspruch 35, **dadurch gekennzeichnet, dass** der Schaft mit der Schlagvorrichtung über ein Verbindungsstück mit dem Arm verbunden ist, welches entlang dem Arm verschiebbar und mittels eines Arretiermittels auf diesem feststellbar ist.

37. Vorrichtung nach Anspruch 36, **dadurch gekennzeichnet, dass** das Verbindungsstück unverdrehbar mit dem Arm verbunden ist, so dass die Werkzeugachse und die Schaftachse parallel bleiben.

38. Vorrichtung nach Anspruch 37, **dadurch gekennzeichnet, dass** die Drehsicherung durch eine Abflachung am Arm erfolgt, während das Verbindungsstück eine entsprechende Abflachung aufweist.

39. Vorrichtung nach einem der Ansprüche 34 bis 38, **dadurch gekennzeichnet, dass** das in den Kopf eingeführte Werkzeug mittels eines Riegels translatorisch verbunden ist und frei drehbar ist, wobei das Werkzeug mit einer Drehmuffe verbunden ist.

40. Vorrichtung nach Anspruch 39, **dadurch gekennzeichnet, dass** die Muffe mit einer Antriebswelle im Eingriff steht, die mittels eines Rads antreibbar ist, um das Werkzeug in Drehung zu versetzen.
